# EUROPEAN PATENT APPLICATION

(11) **EP 2 520 586 A1**
(43) Date of publication of application: **07.11.2012**
(21) Application number: 12164874.5
(22) Date of filing: 29.06.2007
(51) Int. Cl.: C07K 14/755, C12P 21/06, C12N 5/00

(54) **Method of producing factor VIII proteins by recombinant methods**

(30) Priority: 30.06.2006 US 818177 P
(62) Divisional of application: 07812491.4
(71) Applicant: The Regents of the University of Michigan, Ann Arbor, MI 48109-1280 (US); Inspiration Biopharmaceuticals, Inc., Laguna Niguel, CA 92677 (US)
(72) Inventor: Kaufman, Randal J., San Diego, CA 92130 (US); Pipe, Steven W., Ypsilanti, MI 48197 (US); Griffith, Michael J., San Juan Capistrano, CA 92675 (US); Drohan, William N., deceased (US)
(74) Representative: Pallard, Caroline Chantal Patricia

(57) **Abstract**

Provided herein are methods and compositions for producing Factor VIII proteins. Such methods include introducing into a cell a nucleic acid molecule encoding a Factor VIII protein operably linked to a promoter, wherein the promoter is characterized by the ability to produce commercially viable Factor VIII protein; and incubating the cell under conditions for producing commercially viable Factor VIII protein. Also provided are nucleic acid molecules which encode a Factor VIII protein operably linked to a Chinese hamster elongation factor 1-α (CHEF1) promoter, which may be used in the methods provided herein.

## Description

### BACKGROUND OF THE INVENTION

### Related Applications

This Application claims the benefit of priority to U.S. Provisional Application No. 60/818,177, filed June 30, 2006.

### Field of the Invention

Embodiments of the invention relate generally to production of recombinant Factor VIII proteins. Embodiments of the invention also relate to the overexpression or production of recombinant Factor VIII proteins for the treatment of hemophilia A.

### Description of the Related Art

Bleeding disorders can result from a deficiency in the functional levels of one or more of the blood proteins, collectively known as blood coagulation factors, that are required for normal hemostasis, i.e. blood coagulation. The severity of a given bleeding disorder is dependent on the blood level of functional coagulation factors. Mild bleeding disorders are generally observed when the functional level of a given coagulation factor reaches about 5% of normal, but if the functional level falls below 1%, severe bleeding is likely to occur with any injury to the vasculature.

Medical experience has shown that essentially normal hemostasis can be temporarily restored by intravenous infusion of biological preparations containing one or more of the blood coagulation factors. So-called replacement therapy, whereby a biological preparation containing the deficient blood coagulation factor is infused when bleeding occurs (on demand) or to prevent bleeding (prophylactically), has been shown to be effective in managing patients with a wide variety of bleeding disorders. In general, for replacement therapy to be effective, intravenous infusions of the missing coagulation factor are targeted to achieve levels that are well above 5% of normal over a two- to three-day period.

Historically, patients who suffer from hemophilia, a genetically acquired bleeding disorder that results from a deficiency in either blood coagulation Factor VIII (hemophilia A) or Factor IX (hemophilia B), were successfully treated by periodic infusion of whole blood or blood plasma fractions of varying degrees of purity.

More recently, with the advent of biotechnology, biologically active preparations of synthetic (recombinant) blood coagulation factors have become commercially available for treatment of blood coagulation disorders. Recombinant blood coagulation proteins are essentially free of the risks of human pathogen contamination that continue to be a concern that is associated with even high purity commercial preparations that are derived from human blood.

Adequate treatment of bleeding disorders is largely limited to the economically-developed regions of the world. In the case of hemophilia it is estimated that over 75% of the patient population worldwide receives inadequate or, worse, no treatment of their disease. For many regions of the world, the cost of safe and effective commercial preparations of coagulation factors is prohibitive for routine management of bleeding disorders and, in some cases, only emergency treatment with donated products is available.

In regions of the world where adequate treatment of bleeding disorders is potentially available, the cost is very high and patients are almost always dependent on third party payors, e.g. health insurance or government subsidized programs, to acquire the commercial products needed. On average, hemophilia treatment in the United States is estimated to cost about $50,000 per patient per year for the commercial product required for routine, on-demand, care. However, this cost could be much higher insofar as the Medical and Scientific Advisory Committee for the National Hemophilia Foundation has recommended that patients should receive prophylactic treatment which, in the case of an adult hemophiliac, could drive the annual cost to well over $250,000 per year. Given that life-time insurance caps of about $1 million are generally associated with most policies in the United States, hemophiliacs are severely constrained in terms of the amount of commercial product that they can afford for care which, at the least, affects their quality of life during adulthood and, at the worst, raises the risk of life-threatening bleeding.

For the past 25 years or so, biotechnology has offered the promise of producing low cost biopharmaceutical products. Unfortunately, this promise has not been met due in major part to the inherent complexity of naturally occurring biological molecules and a variety of limitations associated with the synthesis of their recombinant protein counterparts in genetically engineered cells. Regardless of the cell type, e.g. animal, bacteria, yeast, insect, plant, etc., that is chosen for synthesis, proteins must achieve certain minimal structural properties for safe and effective therapeutic use. In some cases, recombinant proteins must simply fold correctly after synthesis to attain the three-dimensional structure required for proper function. In other cases, recombinant proteins must undergo extensive, enzyme directed, post-translational modification after the core protein has been synthesized within the cell. In addition, protein made in foreign recombinant cells must be successfully secreted out of the cell. Deficiencies in any one of a number of intracellular trafficking or enzymatic activities can result in the formation of a large percentage of non-functional protein and limit the usefulness of a genetically engineered cell system for the economical production of a biopharmaceutical product intended for commercial use.

Achieving high levels of functional Factor VIII proteins by recombinant technology has been limited in part by the lack of availability of suitable Factor VIII expression systems. Attempts by others at overexpressing Factor VIII at levels required to produce commercially viable Factor VIII have failed. To increase the availability of blood coagulation Factor VIII protein to meet the worldwide medical need for the treatment of bleeding disorders such as hemophilia A, improvements in the production of fully functional protein, Factor VIII, from genetically engineered cells is required. New recombinant expression systems capable of producing large quantities of functional Factor VIII are needed. Because wild-type Factor VIII is secreted at relatively low levels in transfection studies, it would further be desirable to provide expression systems capable of producing large quantities of Factor VIII protein having increased secretion as compared to wild-type Factor VIII. The present application addresses a need for a method to produce recombinant Factor VIII protein in sufficient yield for commercial production.

Further aspects, features and advantages of this invention will become apparent from the detailed description of the embodiments which follow.

### SUMMARY OF THE INVENTION

Provided herein are methods for overexpressing or producing a recombinant Factor VIII protein. The provided methods comprise introducing into a cell a nucleic acid molecule encoding a Factor VIII protein operably linked to a promoter, wherein the promoter is characterized by the ability to produce commercially viable Factor VIII protein; and incubating the cell under conditions for overexpressing or producing Factor VIII protein. The cell used for recombinant production of Factor VIII protein may be a mammalian cell and may further be selected from the group consisting of a COS-1, CHO and HEK 293 cell. The nucleic acid molecule operably linked to a promoter may comprise a cDNA which encodes the Factor VIII protein. The promoter operably linked to the nucleic acid molecule encoding a Factor VIII protein may be a Chinese hamster elongation factor 1-α (CHEF1) promoter.

A Factor VIII protein overexpressed or produced by the recombinant methods provided herein may be a wild-type Factor VIII protein which is in one embodiment is a human protein. A Factor VIII protein may comprise modifications that enhance secretion and/or expression of the Factor VIII protein to be overexpressed or produced. Accordingly, the Factor VIII protein may comprise a deletion of the B-domain starting at Arg 740 when the protein is aligned with the wild-type Factor VIII, followed by the addition of an amino acid spacer containing at least one N-linked glycosylation site, wherein the amino acid spacer containing the at least one N-linked glycosylation site facilitates the secretion or expression of the B-domain-deletion Factor VIII protein. The Factor VIII protein may further comprise an amino acid sequence inserted at position 750 when the protein is aligned with wild-type Factor VIII, the inserted amino acid sequence consisting of a 226 amino acid spacer containing 6 N-linked glycosylation sites, thereby partially replacing the B domain of the modified Factor VIII protein. The Factor VIII protein may comprise an amino acid sequence inserted at position 769 when the protein is aligned with the wild-type Factor VIII, the inserted amino acid sequence consisting of a 29 amino acid spacer containing one N-linked glycosylation site, thereby partially replacing the B domain of the modified Factor VIII protein. The Factor VIII protein may comprise an amino acid sequence inserted at position 794 when the protein is aligned with the wild-type Factor VIII, the inserted amino acid sequence consisting of a 55 amino acid spacer containing 2 N-linked glycosylation sites, thereby partially replacing the B domain of the modified Factor VIII protein. The Factor VIII protein may comprise an amino acid sequence inserted at position 857 when the protein is aligned with the wild-type Factor VIII, the inserted amino acid sequence consisting of a 117 amino acid spacer containing 3 N-linked glycosylation sites, thereby partially replacing the B domain of the modified Factor VIII protein. The Factor VIII protein may comprise an amino acid sequence inserted at position 903 when the protein is aligned with the wild-type Factor VIII, the inserted amino acid sequence consisting of a 163 amino acid spacer containing 4 N-linked glycosylation sites, thereby partially replacing the B domain of the modified Factor VIII protein. The Factor VIII protein may comprise an amino acid sequence inserted at position 946, the inserted nucleic acid sequence consisting of a 206 amino acid spacer containing 5 N-linked glycosylation sites, thereby partially replacing the B domain of the modified Factor VIII protein. The Factor VIII protein may comprise an amino acid sequence inserted at position 1009 when the protein is aligned with the wild-type Factor VIII, the inserted amino acid sequence consisting of a 269 amino acid spacer containing 8 N-linked glycosylation sites, thereby partially replacing the B domain of the modified Factor VIII protein.

Also provided herein are methods for identifying a cell expressing commercially viable Factor VIII protein, comprising: a) introducing into cells a nucleic acid molecule encoding a Factor VIII protein operably linked to a promoter, wherein the promoter is characterized by the ability to overexpress or produce commercially viable Factor VIII protein; b) incubating the cells under conditions for overexpressing or producing Factor VIII protein; c) selecting clones expressing high levels of FVIII relative to the other clones; d) recloning the cells selected in step c); and e) identifying at least one subclone expressing a higher level of FVIII relative to those selected in step c). This method may further comprise: f) recloning the at least one subclone identified in step e); and g) identifying at least one subclone expressing a higher level of FVIII relative to the at least one subclone selected in step e).

Also provided herein are nucleic acid molecules encoding a Factor VIII protein operably linked to a promoter, wherein the promoter is characterized by the ability to overexpress or produce commercially viable amounts of Factor VIII protein. A nucleic acid molecule may comprise a cDNA which encodes the Factor VIII protein. The promoter operably linked to the nucleic acid molecule may be a Chinese hamster elongation factor 1-α (CHEF1) promoter. The nucleic acid molecule encoding the Factor VIII protein may comprise modifications that enhance secretion and/or expression of the Factor VIII protein to be overexpressed or produced. Accordingly, the nucleic acid molecule may encode a Factor VIII protein comprising a deletion of the B-domain starting at Arg 740 when the protein is aligned with the wild-type Factor VIII, followed by the addition of an amino acid spacer containing at least one N-linked glycosylation site, wherein the amino acid spacer containing the at least one N-linked glycosylation site facilitates the secretion or expression of the B-domain-deletion Factor VIII protein. The Factor VIII protein may comprise an amino acid sequence inserted at position 750, the inserted amino acid sequence consisting of a 226 amino acid spacer containing 6 N-linked glycosylation sites, thereby partially replacing the B domain of the modified Factor VIII protein. The Factor VIII protein may comprise an amino acid sequence inserted at position 769 when the protein is aligned with the wild-type Factor VIII, the inserted amino acid sequence consisting of a 29 amino acid spacer containing one N-linked glycosylation site, thereby partially replacing the B domain of the modified Factor Vill protein. The Factor VIII protein may comprise an amino acid sequence inserted at position 794 when the protein is aligned with the wild-type Factor VIII, the inserted amino acid sequence consisting of a 55 amino acid spacer containing 2 N-linked glycosylation sites, thereby partially replacing the B domain of the modified Factor VIII protein. The Factor VIII protein may comprise an amino acid sequence inserted at position 857 when the protein is aligned with the wild-type Factor VIII, the inserted amino acid sequence consisting of a 117 amino acid spacer containing 3 N-linked glycosylation sites, thereby partially replacing the B domain of the modified Factor VIII protein. The Factor VIII protein may comprise an amino acid sequence inserted at position 903 when the protein is aligned with the wild-type Factor VIII, the inserted amino acid sequence consisting of a 163 amino acid spacer containing 4 N-linked glycosylation sites, thereby partially replacing the B domain of the modified Factor VIII protein. The Factor VIII protein may comprise an amino acid sequence inserted at position 946 when the protein is aligned with the wild-type Factor VIII, the inserted amino acid sequence consisting of a 206 amino acid spacer containing 5 N-linked glycosylation sites, thereby partially replacing the B domain of the modified Factor VIII protein. The Factor VIII protein may comprise an amino acid sequence inserted at position 1009 when the protein is aligned with the wild-type Factor VIII, the inserted amino acid sequence consisting of a 269 amino acid spacer containing 8 N-linked glycosylation sites, thereby partially replacing the B domain of the modified Factor VIII protein.

### BRIEF DESCRIPTION OF THE DRAWINGS

FIG. 1A is a diagram of the wild-type FVIII and FV domain structures;
FIG. 1B is a diagram of the inactivation resistant FVIII of the present invention;
FIG. 2 is a table showing secretion activity of the A-1 mutated FVIII proteins of the present invention compared to wild-type FVIII;
FIG. 3 is a graph showing the thrombin activation of APC resistant FVIII of the present invention and wild-type FVIII;
FIGS. 4A and 4B are photographs of gels showing the expression and thrombin cleavage of the APC resistant FVIII of the present invention;
FIGS. 5A and 5B are photographs of gels showing APC cleavage of the APC resistant FVIII of the present invention;
FIG. 6 is a photograph of a gel showing purified wild-type and APC resistant FVIII of the present invention;
FIGS. 7A and 7B are graphs showing APC-mediated functional inactivation of wild-type and APC resistant FVIII of the present invention;
FIG. 8 is a diagram of the domain structure of the single-chain inactivation resistant FVIII of the present invention;
FIG. 9 is a diagram of the domain structure of the inactivation resistant heterodimer FVIII protein of the present invention;
FIG. 10 is a photograph of a gel showing relative synthesis and secretion levels of the inactivation resistant FVIII of the present invention;
FIG. 11 is a photograph of a gel showing the cleavage patterns of the inactivation resistant FVIII of the present invention;
FIG. 12 is a graph showing the functional activation and inactivation of the inactivation resistant FVIII of the present invention as compared to wild-type FVIII;
FIG. 13 is a graph showing the activation and reduced rate of inactivation of immunoaffinity purified inactivation resistant FVIII of the present invention as compared to wild-type FVIII;
FIG. 14 is a graph illustrating the results of an ELISA assay demonstrating antibody-inducible von Willebrand factor (vWF) binding of the inactivation resistant FVIII of the present invention;
FIG. 15 is a graph illustrating the results of an ELISA assay demonstrating antibody-inducible vWF binding of the inactivation resistant FVIII of the present invention following thrombin activation;
FIG. 16 is a graph illustrating the results of an ELISA assay demonstrating antibody-inducible vWF binding of the inactivation resistant FVIII of the present invention following thrombin activation, and retained FVIII activity;

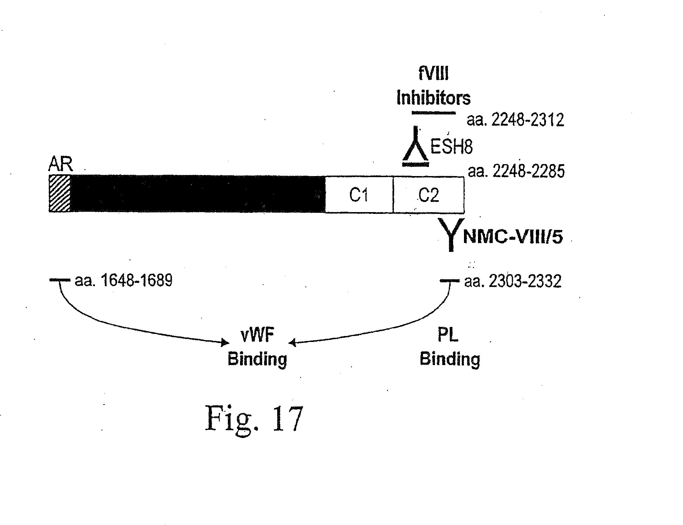

FIG. 17 is a diagram of the FVIII light chain epitopes;

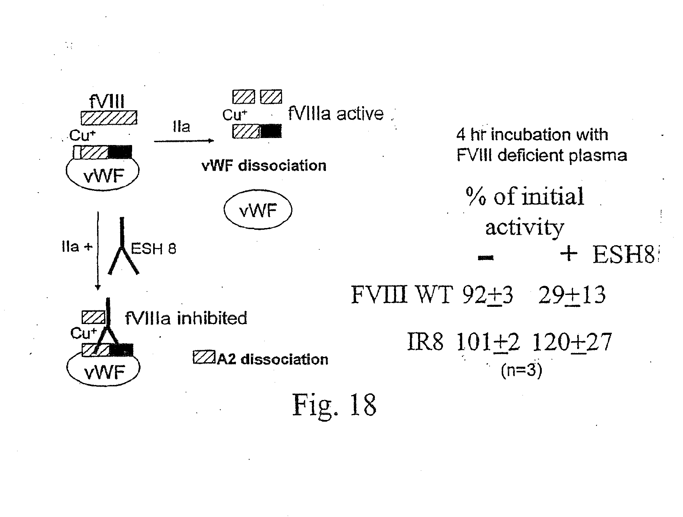

FIG. 18 is a diagram showing that ESH8 does not inhibit inactivation resistant FVIII activity in the presence of vWF;
FIG. 19 is a graph illustrating that thrombin activation of inactivation resistant FVIII/ESH8 does not alter vWF dissociation;
FIG. 20 depicts the kinetics of inactivation resistant FVIII-vWF association and dissociation;
FIGS. 21A and 21B depict the kinetics of thrombin activation;
FIG. 22 depicts the activity of bound FVIII-vWF complexes with and without ESH8;
FIG. 23 is a graph illustrating vWF binding to inactivation resistant FVIII immobilized on Mab NMC-VIII/5;
FIGS. 24A and 24B are graphs illustrating that increasing concentrations of vWF does not inhibit binding of inactivation resistant FVlII/ESH8 complexes to phospholipids;
FIGS. 25A and 25B are graphs illustrating the binding affinity of the inactivation resistant FVIII/ESH8/SPIII complex to phospholipids;
FIGS. 26A and 26B are graphs illustrating that ESH8 increases the half-life of inactivation resistant FVIII in vivo, but in contrast to FVIII WT, does not inhibit activity;
FIG. 27 is a diagram that depicts vWF affinity, PL affinity, and cofactor activity in the presence of vWF for FVIII LC, FVIIIa LC, inactivation resistant FVIII/ESH8 with and without thrombin;
FIG. 28 is a diagram of FVIII B-domain mutants with increasing number of N-linked oligosaccharide content;
FIG. 29 is a graph depicting the relative efficiency of secretion of FVIII B domain variants;
FIG. 30 is a graph that depicts the relative efficiency of secretion of the combined F309S and B domain variant 226aa/N6 ("F309/226aa/N6 variant");
FIG. 31 is a graph that depicts expression of FVIII B domain variants in hemophilia A mice following hydrodynamic tail vein injection of plasmid DNA;
FIG. 32 is a graph that depicts in vivo expression of the FVIII B domain variants in FVIII knockout mice;
FIG. 33 is a graph that depicts FVIII activity over time in mice; and
FIG. 34 depicts the presence of the FVIII B domain variants in cell extract and cell media.

### DETAILED DESCRIPTION OF THE PREFERRED EMBODIMENT

While certain embodiments of the invention are herein described, it is to be understood that modifications will occur to those skilled in the art without departing from the spirit of the invention.

It is the object of the current invention to provide a method for creating a genetically engineered cell that overexpresses or produces a high level of Factor VIII protein in quantities suitable for commercialization world wide. It is a further objective of the invention to provide a Factor VIII molecule which can be given to hemophiliacs by methods other than by intravenous administration.

To overexpress or produce low cost Factor VIII protein biotherapeutics for commercial use on a worldwide basis, a genetically engineered cell must be created for production that (1) overexpresses or produces large quantities of the Factor VIII polypeptide chain that has the desired primary structure and (2) is capable of efficiently performing all of the essential post-translational modifications that are needed to produce a fully functional synthetic biopharmaceutical product.

The term "Factor VIII protein" or "FVIII protein" is intended to encompass a wild-type Factor VIII protein, or any fragment, derivative, modification, or analogue thereof, which encodes a protein, polypeptide, or peptide with the biological activity of Factor VIII. Factor VIII proteins and nucleic acid sequences encoding the same are provided in U.S. Ser. No. 10/383,206, the contents of which are incorporated herein in their entirety by this reference. Examples of Factor VIII proteins include Factor VIII isoform a precursor (NCBI Accession No. NP_000123, the contents of which are incorporated herein by reference) and Factor VIII isoform b precursor (NCBI Accession No. NM_063916, the contents of which are incorporated herein by reference).

As used herein, "biological activity" or "biologically active" is determined with reference to a Factor VIII standard derived from human plasma. Biological activity of a Factor VIII protein may be determined using the commercially available Factor VIII assay, COATEST (Kabi Pharmaceuticals) or other assay in the art. COATEST measures the FVIII-dependent generation of Factor Xa from Factor X, with one unit defined as the amount of FVIII activity in one ml of pooled human plasma, 100 to 200 ng/ml (Vehar et al., Biotechnology of Plasma Proteins, Albertini et al., eds. pg. 2155, Basel, Karger, 1991). Pooled human plasma (George King Bio-Medical, Inc., Overland Park, Kans.) may be used as the FVIII activity standard. The biological activity of the Factor VIII standard is taken to be 100%. In one embodiment, the Factor VIII of the invention has at least 5% of the activity of the Factor VIII standard. In other embodiments, the Factor VIII of the invention has at least 10% of the activity of the Factor VIII standard, at least 15% of the activity of the Factor VIII standard, at least 20% of the activity of the Factor VIII standard, at least 25% of the activity of the Factor VIII standard, at least 30% of the activity of the Factor VIII standard, at least 35% of the activity of the Factor VIII standard, at least 40% of the activity of the Factor VIII standard, at least 45% of the activity of the Factor VIII standard, at least 50% of the activity of the Factor VIII standard, at least 55% of the activity of the Factor VIII standard, at least 60% of the activity of the Factor VIII standard, at least 65% of the activity of the Factor VIII standard, at least 70% of the activity of the Factor VIII standard, at least 75% of the activity of the Factor VIII standard, at least 80% of the activity of the Factor VIII standard, at least 85% of the activity of the Factor VIII standard, or at least 90% of the activity of the Factor VIII standard. "Biologically active" is used interchangably with the term "procoagulant active."

As used herein the term "procoagulant-active" and "active" FVIII, may be used interchangeably to refer to one or more polypeptide(s) or proteins demonstrating procoagulant activity in a clotting assay. The term FVIII may be used herein to encompass FVIIIa and one skilled in the art will appreciate from the context in which the terms are used which term (pre-thrombin activated FVIII or thrombin activated FVIII (FVIIIa)) is intended. As used herein, the term "polypeptides" includes not only full length protein molecules but also fragments thereof which, by themselves or with other fragments, generate FVIII procoagulant activity in a clotting assay. It will be appreciated that synthetic polypeptides of the protein products of the present invention are also within the scope of the invention and can be manufactured according to standard synthetic methods. It will also be appreciated that in the amino acid numbering system used herein, amino acid residue 1 is the first residue of the native, mature FVIII protein. It will further be appreciated that the term "domain" refers to the approximate regions of FVIII, known to those skilled in the art.

The term "DNA sequence encoding a Factor VIII protein" as used herein means DNA which encodes a Factor VIII protein, i.e., such DNA may be a full-length gene encoding a full-length Factor VIII protein, or a truncated gene, or a mutated gene encoding a biologically active Factor VIII protein. The term "DNA sequence" may be a cDNA and refers generally to a polydeoxyribonucleotide molecule and more specifically to a linear series of deoxyribonucleotides connected one to the other by phosphodiester bonds between the 3' and 5' carbons of the adjacent pentoses, or a substantially duplicative sequence thereof. Examples of DNA sequences encoding a Factor VIII protein include genomic Factor VIII DNA (NCBI Accession No. NG_005114, the contents of which are incorporated herein by reference) and cDNA (NCBI Accession No.'s NM_000132 and NM_019863, the contents both of which are incorporated herein by reference). An example of a nucleic acid sequence encoding a Factor VIII protein is a DNA sequence encoding a Factor VIII protein.

The phrase "substantially duplicative" is meant to include those sequences which, though they may not be identical to a given sequence, still result in expression product, proteins, and/or synthetic polypeptides that have FVIII activity in a standard clotting assay. Substantially duplicative sequences include analogs and derivatives thereof.

Factor VIII proteins according to the invention are capable of overexpression or production at a level of at least about 20 IU/mL, at least about 30 IU/mL, at least about 40 IU/mL, at least about 50 IU/mL, at least about 60 IU/mL, at least about 70 IU/mL, at least about 80 IU/mL, at least about 90 IU/mL, at least about 100 IU/mL, at least about 110 IU/mL, at least about 120 IU/mL, at least about 130 IU/mL, at least about 140 IU/mL, at least about 150 IU/mL, at least about 160 IU/mL, at least about 170 IU/mL, at least about 180 IU/mL, at least about 190 IU/mL, at least about 200 IU/mL, or at least about 210 IU/mL of biologically active Factor VIII protein.

As used herein, the term "commercially viable Factor VIII protein" means a Factor VIII protein, which, when overexpressed or produced from tissue culture cells, is capable of overexpression or production at a level of at least about 20 IU/mL, at least about 30 IU/mL, at least about 40 IU/mL, at least about 50 IU/mL, at least about 60 IU/mL, at least about 70 IU/mL, at least about 80 IU/mL, at least about 90 IU/mL, at least about 100 IU/mL, at least about 110 IU/mL, at least about 120 IU/mL, at least about 130 IU/mL, at least about 140 IU/mL, at least about 150 IU/mL, at least about 160 IU/mL, at least about 170 IU/mL, at least about 180 IU/mL, at least about 190 IU/mL, at least about 200 IU/mL, or at least about 210 IU/mL of biologically active Factor VIII protein. Additionally, the term "commercially viable Factor VIII protein" means a Factor VIII protein, which, when overexpressed or produced from tissue culture cells, is biologically active. In one embodiment, the commercially viable Factor VIII protein is at least about 10% biologically active, at least about 15% biologically active, at least about 20% biologically active, at least about 25% biologically active, at least about 30% biologically active, at least about 35% biologically active, at least about 40% biologically active, at least about 45% biologically active, at least about 50% biologically active, at least about 55% biologically active, at least about 60% biologically active, at least about 65% biologically active, at least about 70% biologically active, at least about 75% biologically active, at least about 80% biologically active, at least about 85% biologically active, or at least about 90% biologically active.

The term "processing factor" is a broad term which includes any protein, peptide, non-peptide cofactor, substrate, or nucleic acid molecule which promotes the formation of a functional Factor VIII protein.

An object of the present invention is a genetically engineered CHO or other cell that overexpresses or produces large quantities of Factor VIII proteins whereby the percentage of fully functional protein is adequate to produce a low cost biopharmaceutical product for commercial use on a worldwide basis.

A nucleic acid molecule encoding a Factor VIII protein may be introduced into a cell via transfection. Many transfection methods to create genetically engineered cells that express large quantities of recombinant proteins are well known. Monoclonal antibodies, for example, are routinely manufactured from genetically engineered cells that express protein levels in excess of 1000 IU/mL. The present invention is not dependent on any specific transfection method that might be used to create a genetically engineered cell.

Many expression vectors can be used to create genetically engineered cells. Some expression vectors are designed to express large quantities of recombinant proteins after amplification of transfected cells under a variety of conditions that favor selected, high expressing, cells. Some expression vectors are designed to express large quantities of recombinant proteins without the need for amplification under selection pressure. The present invention is not dependent on the use of any specific expression vector.

To create a genetically engineered cell to overexpress or produce large quantities of a given Factor VIII protein, an expression vector that contains the cDNA encoding the Factor VIII protein is introduced into cells such as by transfection. The present invention requires that a transfected cell is created that is capable, under optimized growth conditions, of overexpressing or producing a minimum of 20 IU/mL of the target Factor VIII protein. Higher levels of production of the target Factor VIII protein may be achieved and could be useful in the present invention. However, the optimum level of overexpression or production of the target Factor VIII protein is a level at or above 20 IU/mL that can be obtained in a significantly increased functional form when the target protein is expressed with selected co-transfected enzymes that cause proper post-translational modification of the target protein to occur in a given cell system.

To create a genetically engineered cell that is capable of efficiently performing all of the essential post-translational modifications that are needed to produce a fully functional synthetic biopharmaceutical product, selected enzymes may be co-introduced along with the Factor VII protein.

The method of the present invention involves the first selection of a cell that may be genetically engineered to overexpress or produce large quantities of a Factor VIII protein.

The cell may be selected from a variety of sources, but is otherwise a cell to which an expression vector containing a DNA may be introduced, which in one embodiment is a cDNA of a Factor VIII gene, or a substantially duplicative sequence thereof.

From a pool of transfected cells, clones are selected that overexpress or produce quantities of the Factor VIII protein over a range (Target Range) that extends from the highest level to the lowest level that is minimally acceptable for the production of a commercial product. Cell clones that overexpress or produce quantities of the Factor VIII protein within the Target Range may be combined to obtain a single pool or multiple sub-pools that divide the clones into populations of clones that produce high, medium or low levels of the Factor VIII protein within the Target Range.

It is considered to be within the scope of the present invention that recombinant cells that produce a Factor VIII protein within the Target Range may be analyzed to determine the extent to which fully functional protein is overexpressed or produced. Such analysis will provide insight into the specific enzyme deficiencies that limit the production of fully functional protein. Further, it is anticipated that analysis of sub-pools consisting of cell clones that overexpress or produce high, medium, or low levels of the Factor VIII protein within the Target Range will provide insight into the specific enzyme deficiencies that limit the overexpression or production of fully functional protein at varying levels of production of the Factor VIII protein. Such analysis, whether done on a single pool of cell clones or on sub-pools, might reveal the specific enzyme deficiencies that must be eliminated to produce fully functional protein.

To eliminate the enzyme deficiencies within a pool of recombinant clones that limit the overexpression or production of fully functional Factor VIII protein within the Target Range, the method of the present invention provides for the transfection of the pool of cells with an expression vector containing a nucleic acid molecule, which may be a cDNA for a protein that, when expressed by a cell clone, will mitigate the enzyme deficiency in whole or in part. It is further contemplated that more than one enzyme deficiency must be mitigated or that mitigation of a deficiency in post-translational modification of the Factor VIII protein requires the presence of the activities of more than one enzyme or protein or other processing factor that may be provided in the method of the present invention by the simultaneous or subsequent (sequential) transfection of the cell clones with additional expression vectors containing cDNA for given proteins.

It is the object of the present invention to provide a method to identify the minimum protein transfection requirements to obtain a high percentage of fully functional Factor VIII protein from a cell clone that overexpresses or produces the Factor VIII protein in a quantity within the Target Range.

In the method of the present invention pools of cell clones that overexpress or produce a Factor VIII protein within the Target Range are subsequently transfected to provide a specific protein or multiple proteins in various combinations. Transfected pools of cell clones are then analyzed to determine the relative percentages of fully functional Factor VIII protein that are now produced by transfectant pools that co-express the various proteins. The transfectant pool that overexpresses or produces the highest percentage of fully functional Factor VIII protein with the minimum number of co-expressed proteins, is selected for subsequent cloning.

In the method of the present invention, the selected transfectant pool is cloned to determine the optimal level of production of fully functional Factor VIII protein that is attained by co-expression of additional protein(s). It is contemplated that higher percentages of fully functional Factor VIII protein will be produced by cell clones that produce lower total amounts of the Factor VIII protein within the Target Range. On the other hand, some cell clones may be superproducers of Factor VIII protein without significant improvements in post translational processing. Nevertheless, such superproducer lines overexpress or produce usable amounts of functional protein as the overall production level is high. The optimal level of production will be the highest level of functional Factor VIII protein.

The practice of the present invention employs, unless otherwise indicated, conventional techniques of molecular biology, microbiology, recombinant DNA, and immunology, which are within the skill of the art. Such techniques are explained fully in the literature. See, e.g., Sambrook, et al., "Molecular Cloning; A Laboratory Manual", 2nd ed (1989); "DNA Cloning", Vols. I and II (D. N Glover ed. 1985); "Oligonucleotide Synthesis" (M. J. Gait ed. 1984); "Nucleic Acid Hybridization" (B. D. Hames & S. J. Higgins eds. 1984); "Transcription and Translation" (B. D. Hames & S. J. Higgins eds. 1984); "Animal Cell Culture" (R. I. Freshney ed. 1986); "Immobilized Cells and Enzymes" (IRL Press, 1986); B. Perbal, "A Practical Guide to Molecular Cloning" (1984); the series, Methods in Enzymology (Academic Press, Inc.), particularly Vols. 154 and 155 (Wu and Grossman, and Wu, eds., respectively); "Gene Transfer Vectors for Mammalian Cells" (J. H. Miller and M. P. Calos eds. 1987, Cold Spring Harbor Laboratory); " Immunochemical Methods in Cell and Molecular Biology", Mayer and Walker, eds. (Academic Press, London, 1987); Scopes, "Protein Purification: Principles and Practice", 2nd ed. 1987 (Springer-Verlag, N.Y.); and "Handbook of Experimental Immunology" Vols I-IV (D. M. Weir and C. C. Blackwell eds 1986). All patents, patent applications, and publications cited in the background and specification are incorporated herein by reference.

### Genetic Engineering Techniques

The production of cloned genes, recombinant DNA, vectors, transformed host cells, proteins and protein fragments by genetic engineering is well known. See, e.g., U.S. Pat. No. 4,761,371 to Bell et al, at Col. 6 line 3 to Col. 9 line 65; U.S. Pat. No. 4,877,729 to Clark et al. at Col. 4 line 38 to Col. 7 line 6; U.S. Pat. No. 4,912,038 to Schilling at Col. 3 line 26 to Col. 14 line 12; and U.S. Pat. No. 4,879,224 to Wallner at Col. 6 line 8 to Col. 8 line 59.

A vector is a replicable DNA construct. Vectors are used herein either to amplify DNA encoding a Factor VIII Protein and/or to express DNA which encodes a Factor VIII Protein. An expression vector is a replicable DNA construct in which a DNA sequence encoding a Factor VIII protein is operably linked to suitable control sequences capable of effecting the expression of a Factor VIII protein in a suitable host. The need for such control sequences will vary depending upon the host selected and the transformation method chosen. Generally, control sequences include a transcriptional promoter, an optional operator sequence to control transcription, a sequence encoding suitable mRNA ribosomal binding sites, and sequences which control the termination of transcription and translation.

In one embodiment, a suitable control sequence comprises a promoter for the elongation factor -1α from Chinese hamster (CHEF1) to provide high level expression of a Factor VIII coagulation factor and/or processing factor(s). The CHEF1 vector is used as described in Deer, et al.(2004) "High-level expression of proteins in mammalian cells using transcription regulatory sequences from the Chinese Hamster EF-1α gene" Biotechnol. Prog. 20: 880-889 and in U.S. Patent No. 5,888,809, both of which are incorporated herein by reference. The CHEF1 vector utilizes the 5' and 3' flanking sequences from the Chinese hamster EF-1α. The CHEF1 promoter sequence includes approximately 3.7 kb DNA extending from a Spel restriction site to the initiating methionine (ATG) codon of the EF-1α protein. The DNA sequence is set forth in SEQ ID NO: 1 of U.S. Patent No. 5,888,809.

Amplification vectors do not require expression control domains. All that is needed is the ability to replicate in a host, usually conferred by an origin of replication, and a selection gene to facilitate recognition of transformants.

Vectors comprise plasmids, viruses (e.g., adenovirus, cytomegalovirus), phage, and integratable DNA fragments (i.e., fragments integratable into the host genome by recombination). The vector replicates and functions independently of the host genome, or may, in some instances, integrate into the genome itself. Expression vectors should contain a promoter and RNA binding sites which are operably linked to the gene to be expressed and are operable in the host organism.

DNA regions are operably linked or operably associated when they are functionally related to each other. For example, a promoter is operably linked to a coding sequence if it controls the transcription of the sequence; or a ribosome binding site is operably linked to a coding sequence if it is positioned so as to permit translation.

Transformed host cells are cells which have been transformed or transfected with one or more Factor VIII protein vector(s) constructed using recombinant DNA techniques.

### Factor VIII proteins

A nucleic acid molecule operably linked to a promoter of the present invention encodes a protein comprising any Factor VIII protein. Examples of Factor VIII proteins and nucleic acid molecules encoding the same are described in U.S. Ser. No. 10/383,206, the contents of which are incorporated herein in their entirety by this reference.

Purified and isolated nucleic acid sequences encoding FVIII are herein provided for use in conjunction with the invention. Nucleic acid sequences encoding amino acid sequences corresponding to known human FVIII sequences, that include an A1-domain mutation are provided. More specifically, nucleic acid sequences are provided that encode amino acid sequences corresponding to known human FVIII sequences wherein amino acid residue 309, phenylalanine, is mutated. In one embodiment, Phe309 is either deleted or substituted with any other amino acid residue, such as serine. In another embodiment, the human FVIII sequences are B-domain deleted (BDD-FVIII). The resulting FVIII protein is capable of secretion at levels higher than typically obtained with wild-type FVIII and retains procoagulant activity.

In another embodiment, the nucleic acid sequences of the present invention encode FVIII B-domain mutants, wherein a portion of the B-domain is deleted. In particular, it has been shown that the addition of N-linked glycosylation sites can improve the secretion of BDD-FVIII up to 10-fold, as well as increase FVIII expression in vivo. In one embodiment, the nucleic acid sequences of the present invention encode FVIII B domain mutants, wherein the B domain is truncated i.e., the BBD-FVIII includes increasing segments from the amino-terminal end of the B domain. In one embodiment, increasing segments from the amino-terminal end by 29 amino acids demonstrated a 1.7-fold improved secretion of BDD-FVIII. In yet another embodiment, increasing segments from the amino-terminal end of the B domain by 54 amino acids demonstrated a 3.4-fold improved secretion of BDD-FVIII. In still another embodiment, increasing segments from the amino-terminal end of the B domain by 117 amino acids demonstrated a 5.3-fold improved secretion of BDD-FVIII. In a further embodiment, increasing segments from the amino-terminal end of the B domain by 163 amino acids demonstrated a 8.5-fold improved secretion of BDD-FVIII. In yet another embodiment, increasing segments from the amino-terminal end of the B domain by 226 amino acids demonstrated a 10.8-fold improved secretion of BDD-FVIII. It has thus been found that the FVIII B-domain mutants of the present invention show increased secretion proportionate to their N-linked oligosaccharide content.

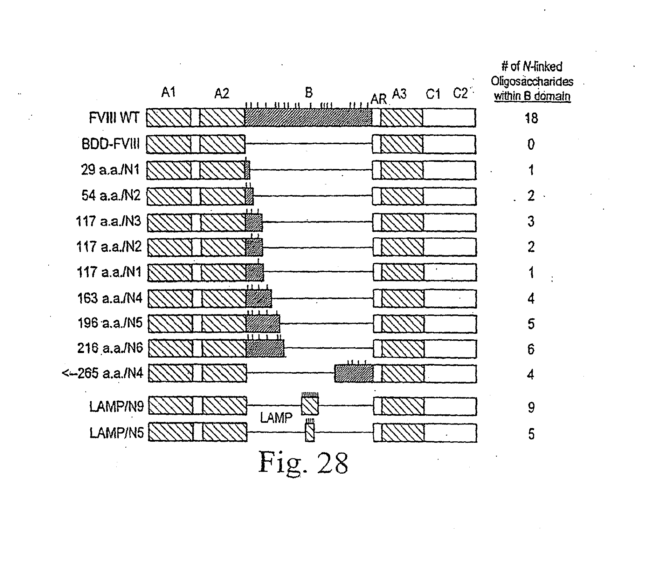

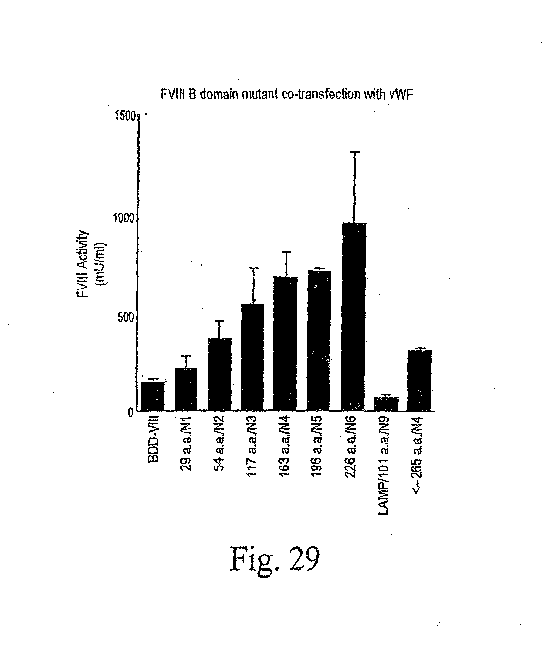

In a further embodiment, the nucleic acid sequences of the present invention encode a hybrid FVIII molecule, which includes a FVIII B-domain mutant and the Phe309 mutant, as described herein. In one embodiment, the FVIII B-domain mutant comprises 226 amino acids at the amino-terminal end of the B-domain (also referred to herein as the "b226N6 B domain variant" which includes 6 consensus sites for N-linked glycosylation, see FIGS. 28 and 29). This embodiment, yields superior expression and activity as compared to either mutation alone.

In a further embodiment, the secretion efficiency of a FVIII B-domain mutant comprises 226 amino acids at the amino-terminal end of the B domain and includes 6 consensus sites for N-linked glycosylation (also referred to herein as the "226N6 variant" or "226aa/N6 variant") and is further enhanced with the point mutation F309S. The combined F309S and B domain 226aa/N6 variant is also referred to herein as the "F309/226aa/N6 variant" or "309S/226aa/N6."

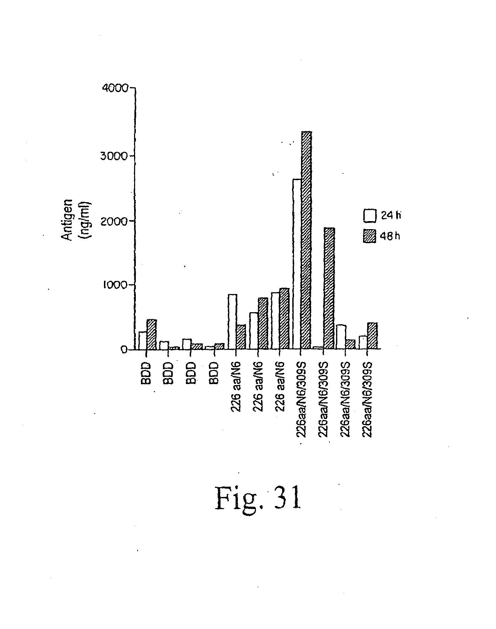

In yet another embodiment, FVIII with minimal B domain content can provide more efficient expression in vitro and in vivo (FIG. 31).

Nucleic acid sequences encoding amino acid sequences corresponding to known human FVIII sequences containing mutated APC cleavage sites are also provided. In one embodiment, the APC cleavage sites Arg336 and Arg562 are mutated, such as to isoleucine and lysine, respectively (R3361 and R562K). The resulting FVIII protein is APC resistant.

Nucleic acid sequences are also provided which encode amino acid sequences corresponding to known human FVIII sequences, wherein the B-domain is deleted, the von Willebrand factor binding site (i.e., the acidic region of the amino terminus of the light chain) is deleted, a thrombin cleavage site is mutated, and an amino acid sequence spacer is inserted between the A2- and A3-domains. This embodiment may further include an APC cleavage site mutation, for example one or both of the APC cleavage site mutations described herein. In one embodiment, the thrombin cleavage site Arg740 is mutated, such as by substitution with alanine (R740A) or lysine (R740K). The amino acid sequence spacer is of a sufficient length to allow the protein to be activated by thrombin to achieve a heterodimer, wherein the A2-domain remains covalently associated with the light chain. In one embodiment, the spacer is approximately 54 residues in length. In another embodiment, the spacer comprises the 54 residues of the amino portion of the wild-type FVIII B-domain, i.e. residues 741 to 794, wherein residue 794 is threonine or leucine. The single-chain polypeptide upon activation with thrombin, becomes a heterodimer, having an approximate five-fold increase in specific activity compared to purified wild-type FVIII.

In a further embodiment, the inactivation resistant FVIII of the present invention may be employed in combination with an antibody or cross-linking agent which increases the protein's binding affinity to vWF. For example, when the vWF binding site-deleted inactivation resistant FVIII of the present invention is in the presence of ESH8, a commercially available mouse monoclonal antibody (American Diagnostics, Inc. Greenwich, Conn.), which recognizes an epitope at amino acids 2248 to 2285 within the C2-domain, the inactivation resistant FVIII binds to vWF. As set forth in greater detail in Example 4, the inactivation resistant FVIII of the present invention has at least a 10-fold reduced affinity for vWF compared to wild-type FVIII, however, in the presence of ESH8, it has only a 2-fold reduced affinity for vWF. It has recently been reported that ESH8 can function as an inhibitor of wild-type FVIII activation by increasing the affinity of thrombin-cleaved FVIII (FVIIIa) for vWF. Saenko, E. L. et al., Blood 86, Abstract No. 749 (1995). By delaying the release of FVIIIa from vWF, A2 dissociation and further proteolytic cleavages likely inactivate the FVIIIa before it can fully release from vWF and exert its cofactor function. A human inhibitor antibody that recognizes an epitope at amino acids 2218 to 2307 within the C2-domain has also been reported that appears to inhibit wild-type FVIII activation by a similar mechanism and may similarly be used to induce vWF binding. Shima, M. et al., Blood 86, Abstract No. 748 (1995) and Shima, M. et al., British J. Hematol. 91: 714-721 (1995).

In yet a further embodiment, the nucleic acid sequences of the present invention encode APC resistant FVIII described herein, having an additional mutation at Phe309. In one embodiment, Phe309 is deleted or substituted with another amino acid, e.g., serine. The nucleic acid sequences of the present invention may also encode inactivation resistant FVIII described herein, also having an additional mutation at Phe309. Again, Phe309 is may be deleted or substituted with another amino acid, e.g., serine. It will further be appreciated that the nucleic acid sequences of the present invention may encode APC resistant FVIII and inactivation resistant FVIII amino acid sequences having a mutated B-domain, i.e. the addition of N-linked glycosylation sites in an otherwise BDD-FVIII. Thus, the nucleic acid sequences of the present invention encode FVIII proteins that exhibit inactivation resistance and/or increased secretion.

It will be appreciated that due to the increased specific activity of the proteins of the present invention, a lower dosage of protein may be administered to hemophiliac patients while maintaining therapeutically effective FVIII activity levels. In addition to cost savings, by utilizing the proteins of the present invention in FVIII replacement therapy, the total exposure of protein to the patient is reduced, thereby lowering the likelihood of inhibitor formation. It will further be appreciated that the proteins of the present invention are also useful in gene therapy-related treatment. DNA sequences for human FVIII are known, as are expression methods (see, e.g. Toole et al., Nature 312:312-317 (1984); Wood et al., Nature 312:330-337, Vehar et al., Nature 312:337-342, U.S. Pat. No. 4,757,006, WO 87/04187, WO 88/08035 and WO 88/03558). The purified and isolated nucleic acid sequences encoding the FVIII protein of the present invention, i.e. a nucleic acid sequence encoding a polypeptide sequence substantially the same as human FVIII or variants thereof modified as is known in the art and described herein, may be made by conventional techniques. For example, the mutations at Phe309 and the APC and thrombin cleavage sites may thus be made by site-directed mutagenesis of the cDNA. One of skill in the art will recognize that "mutation" refers to any alteration including but not limited to, substitutions, insertions and deletions. It will further be appreciated that the remainder of the FVIII nucleic acid sequence may vary from the wild-type FVIII by containing additional modifications such as those disclosed in U.S. Pat. No. 5,004,803, WO 86/06101, and WO 87/07144. FVIII analogs have been developed to better understand the specific structural requirements for FVIII activatibility, inactivatibility, and in vivo efficacy and are also within the scope of the present invention. Included among the features to be optimized are simplified preparation, ease of administration, stability, improved clearance/distribution characteristics, reduced immunogenicity, and prolonged half-life. Moreover, it will be appreciated that variant FVIII nucleic acid sequences in accordance with the present invention also include allelic variations, i.e. variations in sequence due to natural variability from individual to individual, or with other codon substitutions or deletions which still retain FVIII-type procoagulant activity.

Alternate nucleic acid forms, such as Factor VIII genomic DNA, cDNA, and DNA prepared by partial or total chemical synthesis from nucleotides, as well as DNA with mutations, operably linked to a promoter, are also within the contemplation of the invention.

Association of nucleic acid sequences provided by the invention with homologous or heterologous species expression control sequences, such as promoters, operators, regulators, and the like, allows for in vivo and in vitro transcription to form mRNA which, in turn, is susceptible to translation to provide FVIII proteins and related poly- and oligopeptides in large quantities. The present invention thus comprises the expression products of the nucleic acid sequences of the invention, as well as activated forms of these expression products. In an expression system of the invention, FVIII encoding sequences may be operatively associated with a regulatory promoter sequence allowing for transcription and translation in a mammalian cell to provide, for example, FVIII having clotting activity.

The introduction of the sequences of the present invention into prokaryotic and eukaryotic host cells by standard transformation and transfection processes, potentially involving suitable viral and circular DNA plasmid vectors, is also within the contemplation of the invention. Prokaryotic and eucaryotic cell expression vectors containing and capable of expressing the nucleic acid sequences of the present invention may be synthesized by techniques well known to those skilled in this art. The components of the vectors such as the bacterial replicons, selection genes, enhancers, promoters, and the like, may be obtained from natural sources or synthesized by known procedures (see, e.g. Kaufman et al., J. Mol. Biol. 159:601-621 (1982) and Kaufman, PNAS 82:689-693 (1995)). Expression vectors useful in producing proteins of this invention may also contain inducible promoters or comprise inducible expression systems as are known in the art.

Established cell lines, including transformed cell lines, are suitable as hosts. Normal diploid cells, cell strains derived from in vitro culture of primary tissue, as well as primary explants (including relatively undifferentiated cells such as hematopoietic stem cells) are also suitable. Candidate cells need not be genotypically deficient in the selection gene so long as the selection gene is dominantly acting.

The use of mammalian host cells provides for such post-translational modifications, e.g. proteolytic processing, glycosylation, tyrosine, serine, or threonine phosphorylation, as may be made to confer optimal biological activity on the expression products of the invention. Established mammalian cell lines may be used, e.g. CHO (Chinese Hamster Ovary) cells. Alternatively, the vector may include all or part of the bovine papilloma virus genome (Lusky et al., Cell 36:391-401 (1984)) and be carried in cell lines such as C127 mouse cells as a stable episomal element. Other usable mammalian cell lines include HeLa, COS-1 monkey cells, melanoma cell lines such as Bowes cells, mouse L-929 cells, 3T3 lines derived from Swiss, Balb-c or NIH mice, BHK or HaK hamster cell lines, and the like.

Whichever type of expression vector is used, the FVIII nucleic acids of the present invention may be coexpressed with a nucleic acid sequence encoding von Willebrand factor (vWF) or an analog thereof, e.g. as described in WO 87/06101, WO 88/08035 and U.S. Pat. No. 5,250,421. The protein may be expressed in media containing a protease inhibitor such as aprotinin, e.g. in an amount from about 0.01 to about 5%, or from about 0.5 to about 1.0%, (vol/vol) (Aprot., 15-30 Trypsin inhibitor units (TIU)/ml, Sigma) or corresponding amounts of activity units of other protease inhibitors.

Stable transformants are screened for expression of the procoagulant product by standard immunological or activity assays. The presence of the DNA encoding the procoagulant proteins may be detected by standard procedures such as Southern blotting. Transient expression of the procoagulant genes during the several days after introduction of the expression vector into suitable host cells such as COS-1 monkey cells, is measured without selection by activity or immunologic assay of the proteins in the culture medium. Following the expression of the DNA by conventional means, the protein so overexpressed or produced may be recovered, purified and/or characterized with respect to physicochemical, biochemical and/or clinical parameters, all by known methods.

In a further embodiment, the nucleotide sequences of the present invention may be used in gene therapy applications, e.g. to treat hemophilia caused by deficiency of FVIII. The methods of this invention thus comprise the step of introducing the nucleotide sequences of the present invention into a target cell. In order to effectuate transfer, the nucleotide sequences to be transferred must be associated with a vehicle capable of transducing the target cell. Those skilled in the art will appreciate that such vehicles include known gene therapy delivery systems including, but not limited to, adenoviral, retroviral and adeno-associated viral vectors, as well as liposomes and DNA-protein complexes.

### Expression of multiple proteins

By providing the cell with the necessary enzymes and cofactors to process Factor VIII proteins, higher yields of biologically active Factor VIII proteins are achieved. When adequate levels of fully functional Factor VIII proteins are overexpressed or produced by a recombinant cell, lengthy purification steps designed to remove the useless, partially modified, or unmodified Factor VIII protein from the desired product are avoided. This lowers the production cost and eliminates inactive material that may have undesirable side effects for the patient.

Methods for overrexpressing or producing Factor VIII proteins by co-expression with a processing factor can include the following techniques. First, a single vector containing coding sequences for more than one processing factor and a Factor VIII protein can be inserted into a selected host cell. Alternatively, two or more separate vectors encoding a Factor VIII protein plus one or more other processing factors, can be inserted into a host. Upon culturing under suitable conditions for the selected host cell, the two or more proteins are produced and interact to provide cleavage and modification of the proprotein into the mature protein.

Another alternative is the use of two transformed host cells wherein one host cell expresses the Factor VIII protein and the other host cell expresses one or more processing factor which will be secreted into the medium. These host cells can be co-cultured under conditions which allow expression and secretion or release of the recombinant Factor VIII protein and the co-expressed recombinant polypeptides.

In some instances, it may be desirable to have a plurality of copies, two or more, of the gene expressing the Factor VIII protein in relation to the other genes, or vice versa. This can be achieved in a variety of ways. For example, one may use separate vectors or plasmids, where the vector containing the Factor VIII protein encoding polynucleotide has a higher copy number than the vector containing the other polynucleotide sequences, or vice versa. In this situation, it would be desirable to have different selectable markers on the two plasmids, so as to ensure the continued maintenance of the plasmids in the host. Alternatively, one or both genes could be integrated into the host genome, and one of the genes could be associated with an amplifying gene, (e.g., dhfr or one of the metallothionein genes).

Alternatively, one could employ two transcriptional regulatory regions having different rates of transcriptional initiation, providing for the enhanced expression of either Factor VIII protein or the expression of any of the other processing factor polypeptides, relative to Factor VIII protein. As another alternative, one can use different promoters, where one promoter provides for a low level of constitutive expression of Factor VIII protein, while the second promoter provides for a high level of induced expression of the other products. A wide variety of promoters are known for the selected host cells, and can be readily selected and employed in the invention by one of skill in the art such as CMV, MMTV, SV 40 or SRα promoters which are well known mammalian promoters.

### Host cells

Suitable host cells include prokaryote, yeast or higher eukaryotic cells such as mammalian cells and insect cells. Cells derived from multicellular organisms such as mammals are suitable as host cells for recombinant Factor VIII protein synthesis. Propagation of such cells in cell culture has become a routine procedure (Tissue Culture, Academic Press, Kruse and Patterson, editors (1973)). Examples of useful host cell lines are VERO and HeLa cells, Chinese hamster ovary (CHO) cell lines, and WI138, HEK 293, BHK, COS-7, CV, and MDCK cell lines. Expression vectors for such cells ordinarily include (if necessary) an origin of replication, a promoter located upstream from the DNA encoding Factor VIII protein(s) to be expressed and operatively associated therewith, along with a ribosome binding site, an RNA splice site (if intron-containing genomic DNA is used), a polyadenylation site, and a transcriptional termination sequence. In one embodiment, expression is carried out in Chinese Hamster Ovary (CHO) cells using the expression system of U.S. Patent No. 5,888,809, which is incorporated herein by reference.

The transcriptional and translational control sequences in expression vectors to be used in transforming vertebrate cells are often provided by viral sources. For example, commonly used promoters are derived from polyoma, Adenovirus 2, and Simian Virus 40 (SV40). See. e.g.. U.S. Pat. No. 4,599,308.

An origin of replication may be provided either by construction of the vector to include an exogenous origin, such as may be derived from SV 40 or other viral (e.g. Polyoma, Adenovirus, VSV, or BPV) source, or may be provided by the host cell chromosomal replication mechanism. If the vector is integrated into the host cell chromosome, the latter is often sufficient.

Rather than using vectors which contain viral origins of replication, one can transform mammalian cells by the method of cotransformation with a selectable marker and the DNA for the Factor VIII protein(s). Examples of suitable selectable markers are dihydrofolate reductase (DHFR) or thymidine kinase. This method is further described in U.S. Pat. No. 4,399,216 which is incorporated by reference.

Other methods suitable for adaptation to the synthesis of Factor VIII protein(s) in recombinant vertebrate cell culture include those described in M-J. Gething et al., Nature 293, 620 (1981); N. Mantei et al., Nature 281, 40; A. Levinson et al., EPO Application Nos. 117,060A and 117,058A.

Host cells such as insect cells (e.g., cultured Spodoptera frugiperda cells) and expression vectors such as the baculovirus expression vector (e.g., vectors derived from Autographa californica MNPV, Trichoplusia ni MNPV, Rachiplusia ou MNPV, or Galleria ou MNPV) may be employed in carrying out the present invention, as described in U.S. Pat. Nos. 4,745,051 and 4,879,236 to Smith et al. In general, a baculovirus expression vector comprises a baculovirus genome containing the gene to be expressed inserted into the polyhedrin gene at a position ranging from the polyhedrin transcriptional start signal to the ATG start site and under the transcriptional control of a baculovirus polyhedrin promoter.

Prokaryote host cells include gram negative or gram positive organisms, for example Escherichia coli (E. coli) or Bacilli. Higher eukaryotic cells include established cell lines of mammalian origin as described below. Exemplary host cells are E. coli W3110 (ATCC 27,325), E. coli B, E. coli X1776 (ATCC 31,537), E. coli 294 (ATCC 31,446). A broad variety of suitable prokaryotic and microbial vectors are available. E. coli is typically transformed using pBR322. Promoters most commonly used in recombinant microbial expression vectors include the betalactamase (penicillinase) and lactose promoter systems (Chang et al., Nature 275, 615 (1978); and Goeddel et al., Nature 281, 544 (1979)), a tryptophan (trp) promoter system (Goeddel et al., Nucleic Acids Res. 8, 4057 (1980) and EPO App. Publ. No. 36,776) and the tac promoter (H. De Boer et al., Proc. Natl. Acad. Sci. USA 80, 21 (1983)). The promoter and Shine-Dalgarno sequence (for prokaryotic host expression) are operably linked to the DNA encoding the Factor VIII protein(s), i.e., they are positioned so as to promote transcription of Factor VIII Protein(s) messenger RNA from the DNA.

Eukaryotic microbes such as yeast cultures may also be transformed with Factor VIII Protein-encoding vectors. see, e.g., U.S. Pat. No. 4,745,057. Saccharomyces cerevisiae is the most commonly used among lower eukaryotic host microorganisms, although a number of other strains are commonly available. Yeast vectors may contain an origin of replication from the 2 micron yeast plasmid or an autonomously replicating sequence (ARS), a promoter, DNA encoding one or more Factor VIII proteins, sequences for polyadenylation and transcription termination, and a selection gene. An exemplary plasmid is YRp7, (Stinchcomb et al., Nature 282, 39 (1979); Kingsman et al., Gene 7, 141 (1979); Tschemper et al., Gene 10, 157 (1980)). Suitable promoting sequences in yeast vectors include the promoters for metallothionein, 3-phosphoglycerate kinase (Hitzeman et al., J. Biol. Chem. 255, 2073 (1980) or other glycolytic enzymes (Hess et al., J. Adv. Enzyme Reg. 7, 149 (1968); and Holland et al., Biochemistry 17, 4900 (1978)). Suitable vectors and promoters for use in yeast expression are further described in R. Hitzeman et al., EPO Publn. No. 73,657.

Cloned genes of the present invention may code for Factor VIII proteins of any species of origin, including mouse, rat, rabbit, cat, porcine, and human. Nucleic acid molecules encoding Factor VIII proteins that are hybridizable with DNA encoding for Factor VIII proteins disclosed or incorporated by reference herein is also encompassed. Hybridization of such sequences may be carried out under conditions of reduced stringency or even stringent conditions (e.g., conditions represented by a wash stringency of 0.3M NaCl, 0.03M sodium citrate, 0.1% SDS at 60° C or even 70° C to DNA encoding the Factor VIII protein disclosed herein in a standard in situ hybridization assay. See J. Sambrook et al., Molecular Cloning, A Laboratory Manual (2d Ed. 1989)(Cold Spring Harbor Laboratory)).

As noted above, the present invention provides a method of providing a functional Factor VIII. The strategy may include co-expressing Factor VIII protein along with one or more processing factors in a single host cell. In general, the method comprises culturing a host cell which expresses a Factor VIII protein and/or processing factors; and then harvesting the proteins from the culture. The culture can be carried out in any suitable fermentation vessel, with a growth media and under conditions appropriate for the expression of the Factor VIII protein(s) by the particular host cell chosen. The Factor VIII protein can be collected directly from the culture media, or the host cells lysed and the Factor VIII protein collected therefrom. Factor VIII protein can then be further purified in accordance with known techniques.

As a general proposition, the purity of the recombinant protein overexpressed or produced according to the present invention will be an appropriate purity known to the skilled art worker to lead to the optimal activity and stability of the protein. For example, the recombinant Factor VIII protein may be of ultrahigh purity. In one embodiment, the recombinant protein has been subjected to multiple chromatographic purification steps, such as affinity chromatography, ion-exchange chromatography and/or immunoaffinity chromatography to remove substances which cause fragmentation, activation and/or degradation of the recombinant protein during manufacture, storage and/or use. Illustrative examples of such substances that may be removed by purification include thrombin and vonWillebrand factor; other protein contaminants, such as modification enzymes; proteins, such as hamster and mouse proteins, which are released into the tissue culture media from the production cells during recombinant protein production; non-protein contaminants, such as lipids; and mixtures of protein and non-protein contaminants, such as lipoproteins. Purification procedures for Factor VIII proteins are known in the art.

Factor VIII DNA coding sequences, along with vectors and host cells for the expression thereof, are also provided herein.

Also provided herein are methods for identifying a cell expressing commercially viable Factor VIII protein, comprising: a) introducing into cells nucleic acid molecules encoding a Factor VIII protein operably linked to a promoter, wherein the promoter is characterized by the ability to overexpress or produce commercially viable Factor VIII protein; b) incubating the cells under conditions for overexpressing or producing Factor VIII protein; c) selecting clones expressing high levels of FVIII relative to the other clones; d) recloning the cells selected in step c); and e) identifying at least one subclone expressing a higher level of FVIII relative to those selected in step c). This method may further comprise: f) recloning the at least one subclone identified in step e); and g) identifying at least one subclone expressing a higher level of FVIII relative to the at least one subclone selected in step e). Any Factor VIII protein provided herein may be used in conjunction with these methods.

The invention will be further understood with reference to the following illustrative examples and procedures, which is purely exemplary, and should not be taken as limiting the true scope of the present invention. Example 1 describes the preparation and analysis of the A1-domain mutated FVIII of the present invention. Example 2 describes the preparation and analysis of the APC resistant FVIII of the present invention. Example 3 describes the preparation and analysis of the inactivation resistant FVIII of the present invention. Example 4 describes the characterization of the intermolecular protein-protein interactions stabilizing FVIIIa. Example 5 describes the increase of the plasma stability of FVIIIa *in vivo.* Example 6 describes inducible vWF-binding of the inactivation resistant FVIII of the present invention. Example 7 describes the affinity and activity of inactivation resistant FVIII of the present invention. Example 8 describes the pharmacokinetics and efficacy of the inactivation resistant FVIII and inactivation resistant FVIII/ESH8 complex in animals. Example 9 describes the preparation and analysis of the FVIII B domain mutants of the present invention. Example 10 describes the characterization and analysis of the FVIII B domain mutants of the present invention. Example 11 describes expression of bioengineered FVIII in vivo. Example 12 describes pharmaceutical compositions and methods of use of the FVIII proteins and nucleotide sequences of the present invention.

### EXEMPLIFICATION

### EXAMPLE 1 PREPARATION AND ANALYSIS OF A1-DOMAIN MUTATED FACTOR VIII

A statistical algorithm (Blond-Elguindi, S. et al., Cell 75:717-728 (1993)) was applied to predict the BiP binding potential of 7-mer peptides to the 226-336 region of FVIII (residue 1 is the first amino acid residue of the native, mature FVIII protein). Residues Leu303 to Phe309 were found to have a BiP binding score of +14 where any score over +10 has an extremely high probability of binding BiP. Fay, P.J. et al., J. Biol. Chem. 266:8957-8962 (1991). This region contains a hydrophobic cluster where 7 of 11 amino acid residues are Leu or Phe.

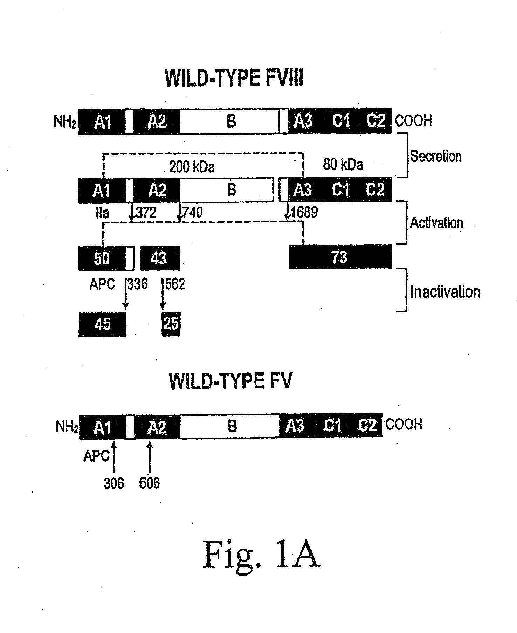

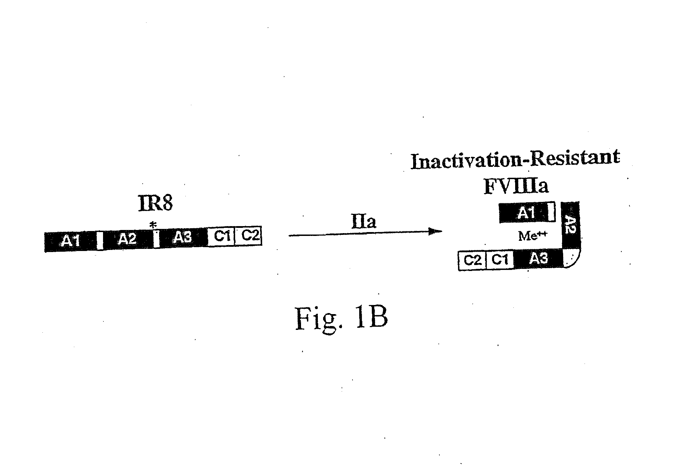

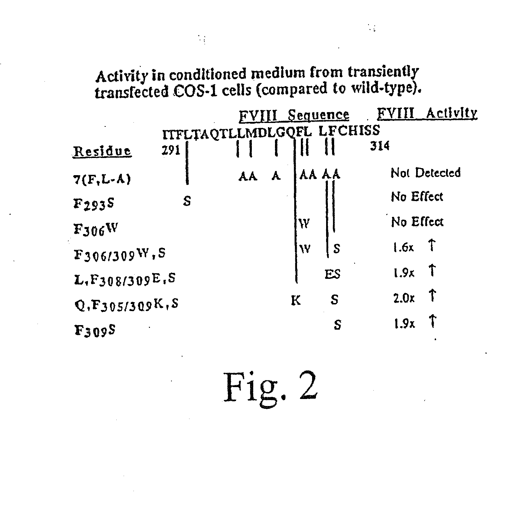

Initially all 7 Leu and Phe residues in the potential BiP binding pocket were mutated to Ala. Site-directed mutagenesis by oligonucleotide overlap-extension polymerase chain reaction (PCR) mutagenesis was utilized. A FVIII/FV chimeric was produced wherein residues 226-336 of FVIII were replaced with the homologous residues from FV (residues 198-313). Marquette, K.A. et al., J. Biol. Chem. 270:10297-10303 (1995). FIG. 1A is a diagram of the wild-type FVIII and FV domain structures. Partially complementary primers that contained the mutation were utilized with two primers directed at the MluI sites at 226 and 336 in the FVIII/FV chimeric cDNA to amplify two overlapping products that contain the directed mutation. These two fragments were isolated and fused together by PCR using the two MluI site containing primers. The resultant MluI fragment was then subcloned into the MluI digested FVIII/FV 226-336 chimera within the expression vector pMT2. All mutations were confirmed by DNA sequencing over the PCR amplified region. Expression vectors encoding these mutants were transfected into COS-1 cells and the conditioned medium taken at 60 hr for analysis of FVIII activity by Coatest activity assay. When all 7 Leu and Phe residues in the potential BiP binding pocket were mutated to Ala, the molecule was not secreted. Subsequently, the Phe residues were individually mutated to the respective amino acid residues in FV. The secretion of the F309S mutants (either alone or in combination with other mutants) were reproducibly increased 2-fold in several transfection experiments. As shown in Figure 2, mutations at other adjacent residues (F293S, F306W) did not improve secretion. The increased secretion of the F309S mutants correlated with a 2-fold increase in FVIII antigen, indicating a specific activity similar to wild-type FVIII. Metabolic labeling with [³⁵S]-methionine for 20 min with a 4 hr chase in medium containing excess unlabeled methionine indicated that the increased secretion of the F309 and Q,F305/309K,S mutants correlated with increased secretion compared to wild-type FVIII,

Stably transfected CHO cell lines were engineered that express the F309S mutant. Of 35 original transfected CHO cell clones selected for dihydrofolate reductase expression, 5 clones were obtained that express significant levels of FVIII (approximately 1 U/ml/10⁶ cells/day). Two of these clones express the same level of FVIII as the original 10A1 cell line that was obtained by screening over 1000 original transfected cell clones. Kaufman, R.J. et al., J. Biol. Chem. 263:6352-6362 (1988). Thus, in low concentrations of methotrexate, the mutation permits high level FVIII expression to be obtained more readily.

Further selection in methotrexate is performed to determine if the maximum productivity of FVIII/cell is improved. Experiments are performed to measure BiP interaction and ATP dependence for secretion for the F309W/S functional FVIII mutant in the stably transfected CHO cells.

### EXAMPLE 2

### PREPARATION AND ANALYSIS OF APC RESISTANT FACTOR VIII Experimental Procedures

*Materials.* FVIII deficient plasma and normal pooled human plasma were obtained from George King Biomedical, Inc. (Overland Park, KS). Monoclonal antibody to the heavy chain of FVIII (F8) coupled to CL4B-sepharose was used and may be prepared by known methods. Activated partial thromboplastin (Automated APTT reagent) was purchased from General Diagnostics Organon Teknika Corporation (Durham, NC). Soybean trypsin inhibitor, phenylmethylsulfonylfluoride (PMSF) and aprotinin were purchased from Boehringer, Mannheim GmbH (Mannheim, Germany). Human á-thrombin was obtained from Sigma Chemical Co. (St. Louis, MO). Human APC was purchased from Enzyme Research Laboratories, Inc. (South Bend, IN). Dulbecco's modified eagle medium (DMEM), á-modification of Eagle's Medium (á-MEM) and methionine-free DMEM were obtained from Gibco BRL (Gaithersburg, MD). Fetal bovine serum was purchased from PAA Laboratories Inc. (Newport Beach, CA).

*Plasmid construction.* Site-directed oligonucleotide-mediated mutagenesis was performed by the gapped-heteroduplex procedure to introduce Arg336lle (R336I) and/or Arg562Lys (R562K) mutations into the FVIII cDNA cloned into the expression vector pED6, as described previously. Pittman, D.D. et al., Method in Enzymology Vol. 222 (San Diego, CA; Academic Press, Inc.) p. 236 (1993)) and Toole, J.J. et al., PNAS (USA) 83:5939 (1986). The mutations were confirmed by extensive restriction endonuclease digestion and DNA sequence analysis. The resultant molecules were designated R3361 or R562K and the double mutant, referred to herein as APC resistant FVIII, was designated R336I/R562K. In addition, a R336I/K338I double mutant was also constructed.

***Analysis* of *synthesis and secretion.*** Plasmid DNA was transfected into COS-1 cells by the diethyl aminoethyl (DEAE)-dextran procedure as described. Pittman, D.D. et al., Method in Enzymology Vol. 222 (San Diego, CA; Academic Press, Inc.) p. 236 (1993). Conditioned medium was harvested 60 hours post transfection in the presence of 10% heat-inactivated fetal bovine serum (FBS) for FVIII assay. Subsequently, cells were metabolically labeled with [³⁵S]-methionine as described before. Pittman, D.D. et al., Method in Enzymology Vol. 222 (San Diego, CA; Academic Press, Inc.) p. 236 (1993). Labeled conditioned medium was harvested and immunoprecipitated with F8 antibody coupled to CL-4B sepharose. Immunoprecipitated proteins from the conditioned medium were washed with PBS containing Triton X-100, resuspended 50 mM Tris-HCl pH 7.5, 150 mM NaCl, 2.5 mM CaCl₂ and 5% glycerol (buffer A), and were treated with or without 8.5 U/ml of thrombin at 37°C for 1 hour. Samples were analyzed by sodium dodecyl sulfate-polyacrylamide gel electrophoresis (SDS-PAGE) under reducing conditions and visualized by autoradiography after fluorography by treatment with En3hance (Dupont; Boston, MA).

***Analysis of APC cleavage of FVIII***. Radiolabeled and immunoprecipitated FVIII was resuspended with buffer A and treated with 30 ìg/ml of bovine APC in the presence of 100 ìg/ml inosithin and 10 mM CaCl₂ at 37°C for 1.5 hr. The resulting polypeptides were separated by SDS-PAGE and visualized by autoradiography as described above.

***Generation of CHO cell lines and purification of FVIII**.* In order to obtain large amounts of FVIII, stably transfected CHO cells lines were engineered containing DNA encoding the wild-type and APC resistant FVIII. The expression plasmids were digested with Cla1 and transfected into CHO cells using the lipofection method. Pittman, D.D. et al., Method in Enzymology Vol. 222 (San Diego, CA; Academic Press, Inc.) p. 236 (1993). Conditioned media were applied to a column of F8 antibody coupled CL-4B sepharose. The bound FVIII was eluted in buffer containing 60% ethylene glycol and concentrated by dialysis against a 10% polyethylene glycol (MW 15K-20K) containing buffer. Fay, P.J. et al., J. Biol. Chem. (in press) (1996). Concentrated samples were dialyzed against modified buffer A containing 5mM CaCl ₂ (buffer B). The FVIII clotting activity of the purified preparations were about 20 U/ml. The structure of purified proteins was evaluated by SDS-PAGE and silver staining (Bio-Rad Laboratories; Hercules, CA).

***FVIII assay.*** FVIII activities were measured in a one stage clotting assay using FVIII deficient plasma as substrate. One unit of FVIII activity is the amount measured in 1 ml of normal human pooled plasma. For thrombin activation, conditioned medium was diluted into buffer A and incubated at room temperature with 1 U/ml thrombin. After incubation for increasing periods of time, aliquots were diluted and assayed for FVIII activity.

***APC inactivation of FVIII**.* Purified FVIII samples diluted to 3 U/ml in buffer B were mixed with 100 ìg/ml inosithin and human APC 100 ng/ml or buffer alone as a control. After increasing periods of time at 37°C, aliquots were diluted and the residual FVIII was determined.

***Effect* of *APC resistant FVIII in the APC resistance assay.*** Twenty U/ml of purified FVIII was diluted with FVIII deficient plasma to 1 U/ml. These samples were tested by the commercialized APC resistance assay kit (Coatest APC Resistance; Chromogenix, Molndal, Sweden) according to the manufacturer.

### Results

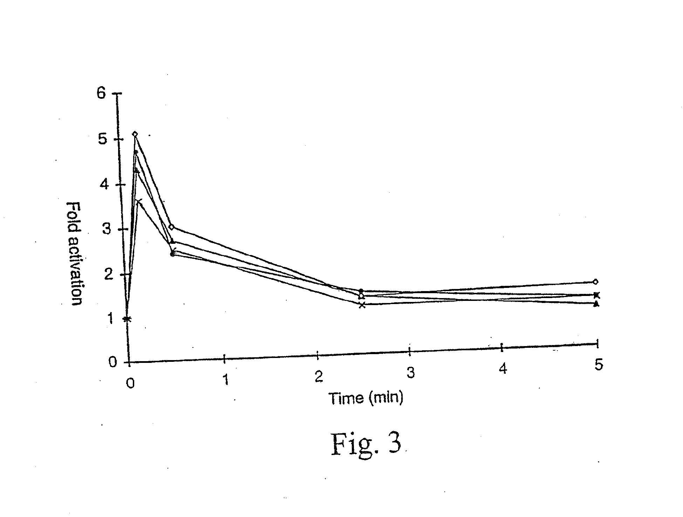

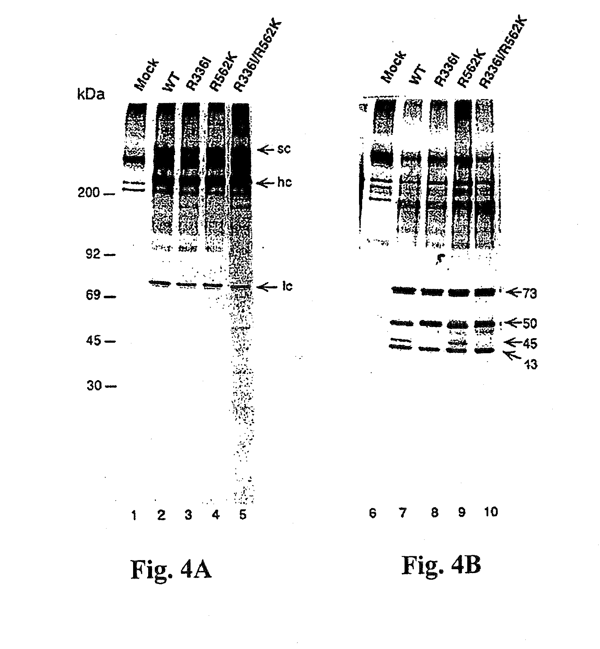

***R336I, R562K, and R3361*/*R562K mutant FVIII molecules are efficiently secreted with FVIII activity similar* to *wild-type FVIII.*** The activity and secretion of FVIII mutants were measured by transient DNA transfection of COS-1 monkey cells. The FVIII clotting activity in the conditioned medium demonstrated that all mutants had FVIII activity similar to wild-type FVIII, approximately 300 mU/ml (see Table 1). Thrombin activation of the conditioned medium samples indicated that there was no difference in the rate of thrombin activation and decay of procoagulant activity. As shown in Figure 3, all samples were immediately activated (3-5 fold) at 10 seconds after thrombin addition and were immediately inactivated. In Figure 3, the symbols represent wild-type FVIII (X), R336I (•), R562K (0) and R336I/R562K ( 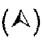 ). To measure FVIII secretion, transfected cells were metabolically labeled with [³⁵S]-methionine for 2 hr and then chased for 4 hr in medium containing excess unlabeled methionine. The secreted proteins were analyzed by immunoprecipitation of labeled conditioned medium. As shown in Figure 4A, wild-type FVIII and all mutants were secreted at similar levels as a 300 kDa single chain and a 200 kDa heavy chain and an 80 kDa light chain. As shown in Figure 4B, thrombin cleavage for all molecules generated the light chain migrating at 73 kDa and the heavy chain derived fragments corresponding to the 50 kDa A1-domain and 43 kDa A2-domain as expected (Figure 4B). In addition, for wild-type FVIII and R562K (Figure 4B, lanes 7 and 9) there was some cleavage at residue 336 to yield a 45 kDa species. In contrast, R336I and R336I/R562K (Figure 4B, lanes 8 and 10) mutants did not generate the 45 kDa species, indicating that isoleucine mutation at residue 336 is resistant to cleavage by excess thrombin. For Figures 4A and 4B, the molecular size markers are shown on the left, "Mock" represents cells that did not receive DNA, and sc, hc and Ic represent single chain, heavy chain and light chain, respectively.

**TABLE 1**

| **FVIII Clotting Activity in Conditioned Medium From Transfected COS-1 Cells** | |
|---|---|
| | **FVIII Clotting Activity (mU/ml) (n=5)** |
| Wild-type | 318.8 ± 36.3 |
| R336I | 306.4 ± 51.2 |
| R562K | 340.0 ± 44.8 |
| R336I/R562K | 308.4 ± 76.9 |

data represents mean ± SD

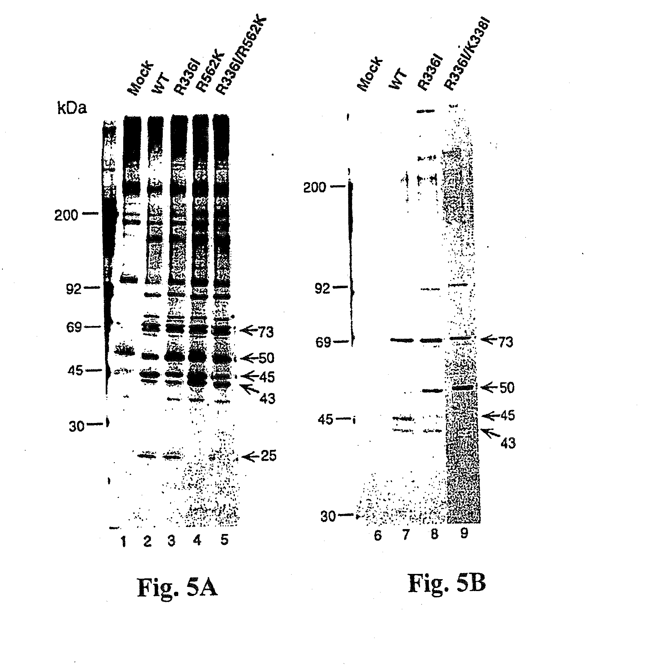

***R562K is completely resistant and R336I is mostly resistant to APC cleavage at the mutated site.*** APC cleavage of FVIIIa was evaluated by treating [³⁵S]-methionine labeled immunoprecipitated FVIII with APC. Analysis of APC cleavage products of wild-type FVIII analyzed by SDS-PAGE on a 5-15% gradient gel detected the heavy chain fragments of 50 kDa and 45 kDa representing the A1-domain, and of 43 kDa representing the A2-domain, that were not present in the conditioned medium of cells that did not receive DNA. As shown in Figure 5A, lane 2, a lower molecular weight product at 25 kDa was detectable, representing the carboxy-terminus of A2-domain. As shown in Figure 5A, lane 3, R336I FVIII was partially resistant to cleavage at residue 336, as indicated by an increase in the 50 kDa and a reduction of the 45 kDa cleavage products compared to wild-type. The R336I displayed no change in the amount of the 25 kDa species indicating efficient cleavage at residue 562. As shown in Figure 5A, lane 4, R562K mutant FVIII was resistant to cleavage at residue 562 as indicated by the increase in the 43 kDa fragment and loss of the 25 kDa fragment. However, the R562K mutant was efficiently cleaved at 336 as indicated by an intense 45 kDa fragment. APC treatment of the R336I/R562K double mutant yielded an increase in the 50 kDa and 43 kDa species, and the reduction of 45 kDa and loss of 25 kDa species compared to wild-type FVIII (see Figure 5A, lane 5). The migration of the 45 kDa fragment derived from APC cleavage of the R336I mutant was slightly reduced upon analysis by SDS-PAGE on an 8% polyacrylamide gel (see Figure 5B, compare lanes 7 and 8). In order to determine whether this mutant may be cleaved at the adjacent lysine at residue 338, an R336I and K338I double mutant was made by site-directed mutagenesis. The R336I/K338I mutant did not generate the 45 kDa fragment upon APC digestion (see Figure 5B, lane 9). In Figures 5A and 5B, molecular size markers are shown on the left and "Mock" represents cells that did not receive DNA.

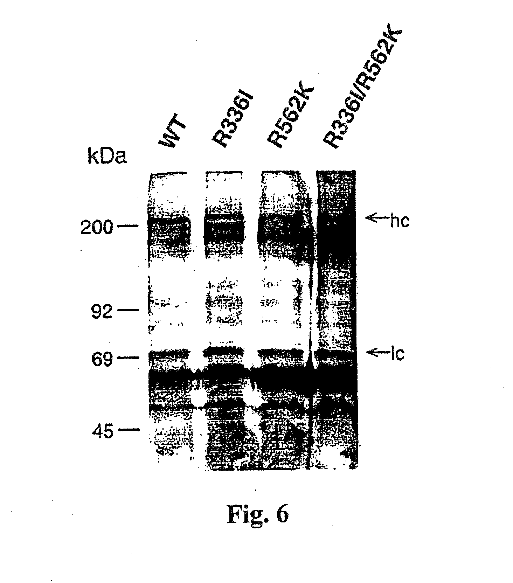

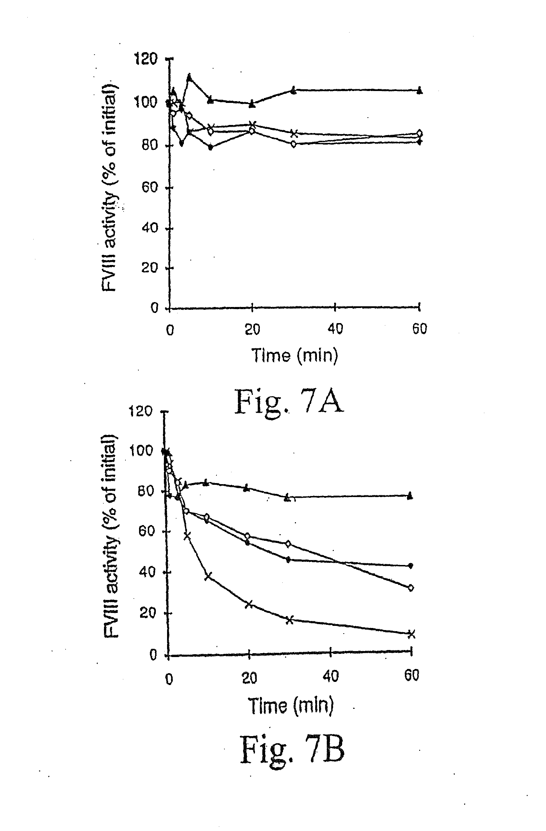

***Mutagenesis at both Arg336 and Arg562 in FVIII are required for resistance to APC inactivation.*** von Willebrand Factor (vWF) inhibits APC inactivation of FVIII. Koedam, J.A. et al., J. Clin. Invest. 82:1236 (1988) and Fay, P.J. et al., J. Biol. Chem. 266:2172 (1991). Therefore, to study APC inactivation, stably transfected CHO cells that express wild-type and the APC cleavage site mutants FVIII molecules were engineered. Conditioned medium was collected for FVIII purification. As shown in Figure 6, analysis of the purified proteins by SDS-PAGE under reducing conditions and silver staining demonstrated that all molecules have similar polypeptide compositions of heavy chain (hc) and light chain (lc) with minimal degradation and absence of vWF. These purified proteins were then analyzed for functional inactivation by APC. As shown in Figure 7A, the activity of all samples, except the R336I/R562K ( 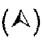 ) double mutant, were reduced to 80% after 10 min incubation at 37°C in the absence of APC and were subsequently stable for 60 min thereafter. In the presence of APC, wild-type FVIII (X) had residual activity of 38% at 10 min and 8% at 60 min. In the presence of APC, the inactivation of R336I (•) and R562K (0) single mutants were similar and both slower than wild-type FVIII. After 60 min 41% and 30% of initial activity remained for the R336i and R562K mutants, respectively. In contrast, the R336I/R562K ( 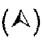 ) double mutant was resistant to inactivation and retained 76% activity after 60 min. The results thus demonstrate that the R336I/R562K double mutant was mostly resistant and both single mutants were only partially resistant to APC inactivation.

***Ability of APC resistance assay kit to detect APC resistant FVIII.*** Presently, a commercially available APC resistance assay kit (Coatest APC Resistance; Chromogenix, Molndal, Sweden) is used to screen the plasma of patients with thrombotic disease associated with the FV R506Q mutation. The ability of this kit to detect APC resistant FVIII was tested by reconstitution of FVIII deficient plasma with either purified wild-type or purified mutant FVIII. The APC resistance ratio was calculated by the measure of the clotting time in the presence of APC divided by the clotting time in the absence of APC (see Table 2). Only the R336I/R562K double mutant demonstrated a lower APC resistance ratio than 2, a value indicative of an APC resistance phenotype. Svensson, P.J. et al., N. Engl. J. Med. 336:517 (1994).

**TABLE 2**

| **APC-Resistance Ratio of Wild-Type FVIII and Mutants in the Commercialized Assay Kit** | |
|---|---|
| | **APC-Resistance Ratio (n=3)** |
| Wild-type | 2.13 ± 0.06 |
| R336I | 2.10 ± 0.00 |
| R562K | 2.13 ± 0.06 |
| R336I/R562K | 1.73 ± 0.06 |

data represents mean ± SD

### Discussion

All mutants were efficiently secreted from COS-1 cells with a FVIII activity similar to wild-type FVIII. Analysis of APC cleavage was performed by [³⁵S]-methionine labeling of protein and analysis of FVIII in the conditioned medium after immunoprecipitation. The R336I mutant was partially resistant to cleavage at residue 336, but was sensitive to cleavage at Arg562. On the other hand, the R562K mutant was completely resistant to cleavage at residue 562, but was sensitive to cleavage at Arg336. These results indicate that either single mutation at Arg336 or Arg562 affects cleavage at the mutated site and that there is not a required order for APC cleavage at these two sites in FVIII. The double mutant R336I/R562K was partially resistant to cleavage at residue 336 and completely resistant at residue 562. The cleavage of R336I likely occurred at an adjacent residue, Lys 338, since a double mutant R336I/K338I was completely resistant to cleavage at this site. These results show that APC cleavage of FVIII can be ragged, *i.e*. it does not have a stringent spacing requirement for cleavage.

Analysis of the kinetics of APC cleavage in FVIII indicated that Arg562 was preferentially cleaved compared to Arg336 and this initial cleavage most closely correlated with the loss of cofactor activity. Fay, P.J. et al., J. Biol. Chem. 266:20139 (1991) The slower inactivation of the R562K single mutant as a consequence of cleavage resistance at residue 562 is consistent with the hypothesis, and that the resultant inactivation was due to cleavage at Arg336. However, the R336I single mutant was only partially inactivated by cleavage at Arg562. It has been shown that both single cleavage site mutants were inactivated at similar rates under the conditions described herein. Assuming that cleavage at Arg336 and Arg562 occur at the same time, the effect of cleavage at either Arg336 or Arg562 for inactivation of FVIII appear to be similar. The rapid inactivation of wild-type FVIII may be due to synergistic roles of cleavage at Arg336 and Arg562 for inactivation of FVIII.

At present, there are no reports describing patients with mutations in the APC cleavage sites of FVIII. To evaluate whether these mutations would have an APC resistance phenotype, the APC resistant FVIII molecules were tested in the commercially available APC resistance assay kit (Coatest APC Resistance; Chromogenix, Molndal, Sweden). Only the R336I/R562K double mutant demonstrated a lower APC-resistance ratio. This assay kit can not therefore, detect either single APC cleavage site mutants of FVIII. In contrast to FVIII, both FV APC single cleavage site mutants, Arg306Gln and Arg506Gln, showed reduced APC-resistance ratios in this assay. The results thus show that the commercially available APC resistance kit will not detect FVIII APC resistant mutants unless both APC cleavages are inhibited.

### EXAMPLE 3

### PREPARATION AND ANALYSIS OF INACTIVATION RESISTANT FACTOR VIII Experimental Procedures

***Materials.*** Anti-heavy chain factor VIII monoclonal antibody (F-8), F-8 conjugated to CL-4B Sepharose and purified recombinant factor VIII protein were obtained from Genetics Institute Inc. (Cambridge, MA). Anti-human vWF horseradish peroxidase(HRP)-conjugated rabbit antibody was obtained from Dako Corp. (Carpinteria, CA). Anti-light chain factor VIII monoclonal antibodies, ESH-4 and ESH-8, were obtained from American Diagnostica, Inc. (Greenwich, CT). Factor VIII-deficient and normal pooled human plasma were obtained from George King Biomedical, Inc. (Overland Park, KS). Activated partial thromboplastin (Automated APTT reagent) and CaCl₂ were obtained from General Diagnostics Organon Teknika Corporation (Durham, NC). Human thrombin, soybean trypsin inhibitor, phenylmethylsulfonylfluoride and aprotinin were obtained from Boehringer, Mannheim GmbH (Mannheim, Germany). O-phenylendiamine dihydrochloride (OPD) was obtained from Sigma Chemical Co. (St. Louis, MO). [³⁵S]-methionine(>1000Ci/mmol) was obtained from Amersham Corp. (Arlington Heights, IL.). En³Hance was obtained from Dupont (Boston, MA). Fetal bovine serum was obtained from PAA Laboratories Inc. (Newport Beach, CA). Dulbecco's modified Eagle's medium (DMEM), methionine-free DMEM, OptiMEM, Biotin N-hydroxy succinimide ester, and streptavidin-horseradish peroxidase conjugate were obtained from Gibco BRL (Gaithersburg, MD).

***Plasmid mutagenesis.*** Mutagenesis was performed within the mammalian expression vector pMT₂(37) containing the FVIII cDNA(pMT₂VIII). Mutant plasmids were generated through oligonucleotide site-directed mutagenesis utilizing the polymerase chain reaction (PCR). For a detailed description of oligonucleotide-directed mutagenesis, see Smith, M., Annu. Rev. Genet. 19:423 (1985).

***Construction 1 - 90*/*73 R740K.*** Vector pMT₂90/73 was used as the DNA template. The 90/73 construct is described in Nesheim, M. et al., J. Biol. Chem. 266: 17815-17820 (1991) and Pittman, D. et al., Blood 70, Abstract No. 392 (1987). Generally, the 90/73 construct is wild-type FVIII cDNA sequence in which the B-domain and the vWF binding site (acidic region of the light chain) have been deleted (del 741-1689). Oligonucleotide-directed mutagenesis was used to create a PCR fragment, KpnI/R740K/Apal, and was ligated into KpnI/ApaI digested pMT₂90/73.

***Construction 2 - 90*/*b*/*73 R740K.*** Vector pMT₂VIII was used as the DNA template. Oligonucleotide-directed mutagenesis was used to create a PCR fragment, KpnI/b/1689 Mlul (where b represents a DNA sequence encoding for amino acid residues 741 to 793 of the wild-type sequence followed by an MluI site predicting amino acids threonine and arginine at residues 794 and 795/1689), which was ligated into KpnI/MluI digested vector pMT₂ VIII/1689/MluI. The following amino acid sequence (and nucleotide sequence encoding same) may be used as an amino acid sequence spacer, wherein residue 794 may be threonine or leucine:

5′ AGC TTC TCC CAG AAT TCA AGA CAC CCT AGC
S F S Q N S R H P S

ACT AGG CAA AAG CAA TTT AAT GCC ACC ACA ATT
T R Q K Q F N A T T I

CCA GAA AAT GAC ATA GAG AAG ACT GAC CCT TGG
P E N D I E K T D P W

TTT GCA CAC AGA ACA CCT ATG CCT AAA ATA CAA
F A H R T P M P K I Q

AAT GTC TCC TCT AGT GAT TTG TTG ATG CTC TTG 3'

N V S S S D L L M L L

***Construction* 3 - *90*/*b*/*73 R740A.*** Vector 90/b/73 was used as the DNA template (wherein b is described above and encodes threonine at residue 794). Oligonucleotide-directed mutagenesis was used to create a PCR fragment, KpnI/R740A/b/ApaI, which was ligated into KpnI/ApaI digested pMT₂90/73.

***Construction 4 - 90*/*b*/*73 R740A*/*R1689A (DM1).*** Vector 90/b/73 R740A was used as the DNA template (wherein b is described above and encodes leucine at residue 794). Oligonucleotide-directed mutagenesis was used to create PCR fragment, KpnI/R740A/b/R1689A/ApaI, which was ligated into Kpnl/Apal digested pMT₂90/73.

***Construction 5 - 90*/*b*/*73 R336I*/*R740A (DM2).*** Vector PMT₂VIII/R336I was digested with Spel and Kpnl. The fragment was ligated into SpeI/KpnI digested 90/b/73 R740A (wherein b is described above and encodes threonine at residue 794).

***Construction* 6 - 90/b/73 *R336I*/*R562K*/*R740A (IR8).*** Vector PMT₂VIII/R562K was digested with BglII and KpnI. The BgIII/R562K/KpnI fragment was ligated into BgIII/KpnI digested 90/b/73 R336I/R740A (wherein b is described above and encodes threonine at residue 794).

The plasmid containing the wild-type FVIII cDNA sequence was designated FVIII WT. All plasmids were purified by centrifugation through cesium chloride and characterized by restriction endonuclease digestion and DNA sequence analysis.

*DNA transfection and analysis.* Plasmid DNA was transfected into COS-1 cells by the DEAE-dextran method. Conditioned medium was harvested at 64 hours post-transfection in the presence of 10% fetal bovine serum. FVIII activity was measured by one-stage APTT clotting assay on a MLA Electra 750. Protein synthesis and secretion were analyzed by metabolically labeling cells at 64 hours post-transfection for 30 minutes with [³⁵S]-methionine (300 mCi/ml in methionine-free medium), followed by a chase for 4 hours in medium containing 100-fold excess unlabeled methionine and 0.02% aprotinin. Cell extracts and conditioned medium containing labeled protein were harvested. WT and mutant FVIII proteins were immunoprecipitated from equal proportions of cell extract and conditioned medium with F-8 coupled to CL-4B Sepharose. Immunoprecipitates were washed and resuspended in Laemmli sample buffer. Samples were analyzed by electrophoresis on a reducing SDS-low bis-8% polyacrylamide gel. The gels were treated with En³Hance and the proteins visualized by autoradiography.

***Protein purification**.* Partially purified IR8 protein was obtained from 200 mls of conditioned medium from transiently transfected COS-1 cells by immunoaffinity chromatography. Partially purified FVIII WT protein was obtained from 200 mls of conditioned medium from stably transfected CHO cells and immunoaffinity purified in the same manner. The proteins eluted into the ethylene glycol-containing buffer were dialyzed and concentrated against a polyethylene glycol (MW ~15-20,000)-containing buffer and stored at -70° C.

*FVIII activity assay.* FVIII activity was measured in a one-stage APTT clotting assay by reconstitution of human FVIII-deficient plasma. For thrombin activation, protein samples were diluted into 50 mM Tris-HCl pH 7.5, 150 mM NaCl, 2.5 mM CaCl₂ and 5% glycerol, and incubated at room temperature with 1 U/ml thrombin. After incubation for increasing periods of time, aliquots were diluted and assayed for FVIII activity. One unit of FVIII activity is the amount measured in 1 ml of normal human pooled plasma.

*FVIII antigen determination.* FVIII antigen was quantified using a sandwich ELISA method utilizing anti-light chain antibodies ESH-4 and ESH-8. Purified recombinant FVIII protein was used as a standard.

### Results

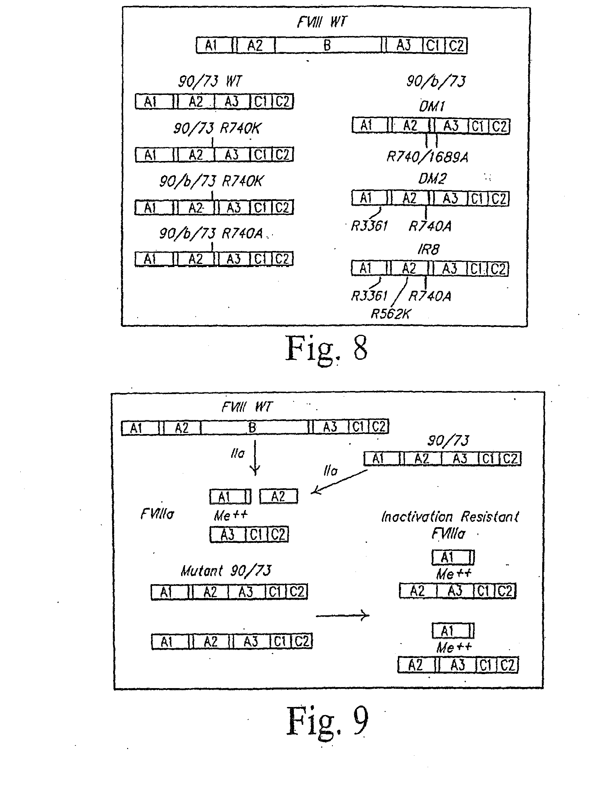

*Generation of FVIII inactivation resistance.* All of the above constructs are based upon 90/73, wherein the B-domain (residues 795 to 1647) and the vWF binding site (residues 1648 to 1688, also referred to as the acidic region of the amino terminus of the light chain), have been deleted. Nesheim, M. et al., J. Biol. Chem. 266: 17815-17820 (1991) and Pittman, D. et al., Blood 70, Abstract no. 392 (1987). Figure 8 sets forth the domain structures of wild-type FVIII and the above constructs as well as the mutations at the APC and thrombin cleavage sites. As described herein and in Figure 8, "b" represents the amino acid sequence spacer which is of a sufficient length to allow the protein to be activated by thrombin to achieve a heterodimer, wherein the A2-domain remains covalently associated with the light chain. In one embodiment, the amino acid sequence spacer is the amino portion of the wild-type B-domain, *i.e.* amino acid residues 741 to 793 followed by an MluI site (for cloning purposes) predicting amino acids threonine or leucine, at residue 794 and arginine at 795/1689.

Figure 8 sets forth a model of activation of the constructs of the present invention. Wild-type FVIII and the mutant 90/73 both achieve a heterotrimer upon thrombin activation. When an amino acid sequence spacer is introduced between the A2- and A3-domains of 90/73 containing a mutation at the thrombin cleavage site (del795-1688/Arg336Iso/Arg562Lys/Arg740Ala), upon activation with thrombin, cleavage only occurs after Arg372, generating a FVIIIa heterodimer. This novel FVIII protein designated IR8, maintains stable activity following thrombin activation.

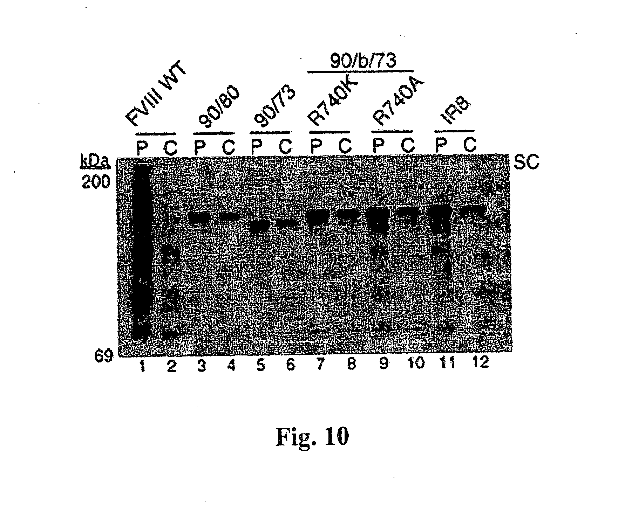

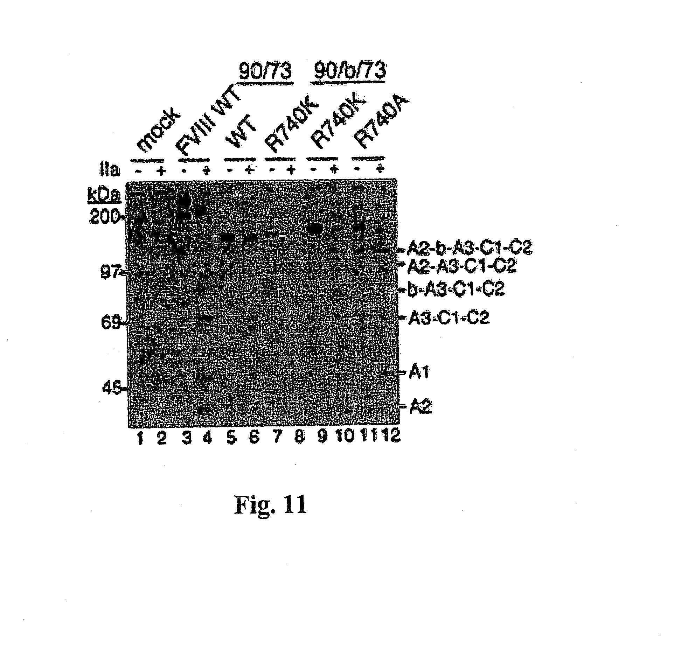

***Synthesis and secretion* of *IR8.*** FVIII WT and the various inactivation-resistance mutants were compared by transient DNA transfection of the cDNA expression vectors into COS-1 monkey cells. At 60 hours following transfection, the rates of synthesis were analyzed by immunoprecipitation of cell extracts from [³⁵S]-methionine pulse-labeled cells. Intracellular FVIII WT was detected in its single chain form and migrated at approximately 250 kDa (Figure 10, lane 1). The mutant 90/80 is a BDD FVIII mutant (del741-1648) previously characterized, that migrates at ~170 kDa and demonstrates an increased intensity from pulse-labeled cell extracts consistent with increased efficiency of synthesis (Figure 10, lane 3). 90/73 migrates slightly faster due to the additional deletion of the residues of the acidic region (Figure 10, lane 5). All the 90/b/73 based constructs including IR8 exhibited similar band intensity to the 90/80 and 90/73 constructs suggesting that the multiple missense mutations did not interfere with efficient protein synthesis. Additional bands within the cell extract are not observed in mock cell extract immunoprecipitated with an anti-FVIII specific antibody and represent both FVIII specific proteins and co-immunoprecipitating intracellular proteins. Following a 4 hour chase period, the majority of FVIII WT is lost from the cell extract (Figure 10, lane 2) and can be recovered from chase conditioned medium in its 280 kDa single chain, 200 kDa heavy chain and 80 kDa light chain forms (Figure 10, lane 3). Although all of the BDD and inactivation-resistance mutants demonstrated significant amounts of their primary translation products remaining within the cell extract following the 4 hour chase (Figure 10, lanes 4, 6, 8, 10, 12), they were all recovered from the chase conditioned medium as single chain species (Figure 11, lanes 5, 7, 9, 11, 13). Therefore the various alterations of the FVIII construct did not have significant impact on secretion.

***Structural stability of IR8 following thrombin cleavage.*** The labeled FVIII proteins immunoprecipated from the chase conditioned medium were incubated with thrombin (1 U/ml) for 30 minutes prior to SDS-PAGE analysis. FVIII WT was efficiently cleaved into a heterotrimer of fragments consisting of a 50 kDa A1 subunit, 43 kDa A2 subunit and 73 kDa thrombin-cleaved light chain, A3-C1-C2 (Figure 11, lane 4). 90/73 WT was also cleaved into a heterotrimer of subunits similar to FVIII WT (Figure 11, lane 6) consistent with previous observations and depicted in Figure 1A. 90/73 Arg740Lys generated a heterodimer of thrombin-cleaved subunits consistent with a 50 kDa A1 subunit and an A2-A3-C1-C2 fused light chain (Figure 11, lane 8). 90/b/73 Arg740Lys demonstrated thrombin cleavage fragments consistent with 2 heteromeric species, a 50 kDa A1/120 kDa A2-b-A3-C1-C2 heterodimer, as well as a 43 kDa A2 subunit and an -85 kDa fragment consistent with a b-A3-C1-C2 fused light chain (Figure 11, lane 10). The appearance of the A2 subunit following incubation with thrombin suggested that Lys740 did not completely abrogate thrombin cleavage in the presence of the b spacer. With the more radical missense mutation to Ala740, a stable heterodimeric species was demonstrated (Figure 11, lane 12). This stable heterodimeric structure following thrombin cleavage was maintained for IR8 with additions of the missense mutations Arg336Iso and Arg562Lys (Figure 11, lane 14).

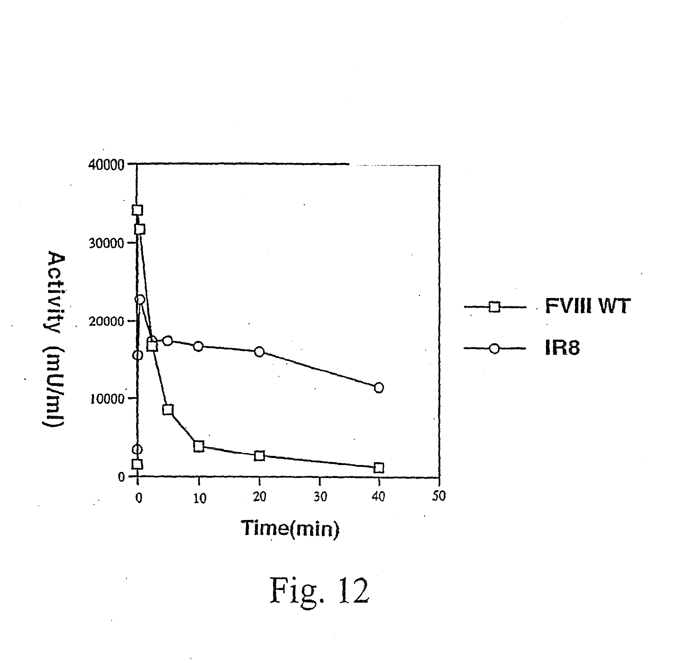

***Functional stability of IR8 following thrombin activation.*** Having demonstrated the structural integrity of the IR8 heterodimer upon thrombin cleavage, the functional consequence of this modification on activation and inactivation was examined in an *in vitro* functional assay. Immunoaffinity purified FVIII WT and IR8 were incubated with thrombin and assayed for FVIII activity by a one stage APTT clotting assay. An example of the functional activation and inactivation is depicted in Figure 12 and is typical of multiple repeat experiments. Under these conditions, FVIII WT was maximally activated within the first 10 seconds of incubation with thrombin, then rapidly inactivated over the next 5 minutes. IR8 did not reach peak activity until 30 seconds incubation with thrombin, suggesting a modestly reduced sensitivity to thrombin activation compared to FVIII WT. In addition, the peak activity for thrombin activated IR8 was lower (74.7 + 6.7% of peak thrombin activated FVIII WT activity, n=3), suggesting some reduced efficiency as a cofactor. However, IR8 demonstrated significant retention of peak activity over the first 10 minutes of incubation with thrombin (66.9 + 5.3% of peak IR8 activity, n=3), a period in which FVIII WT was almost completely inactivated. Although there is a gradual loss of peak IR8 activity with prolonged incubation with thrombin, IR8 still retained ~38% of peak activity after 4 hours incubation with thrombin.

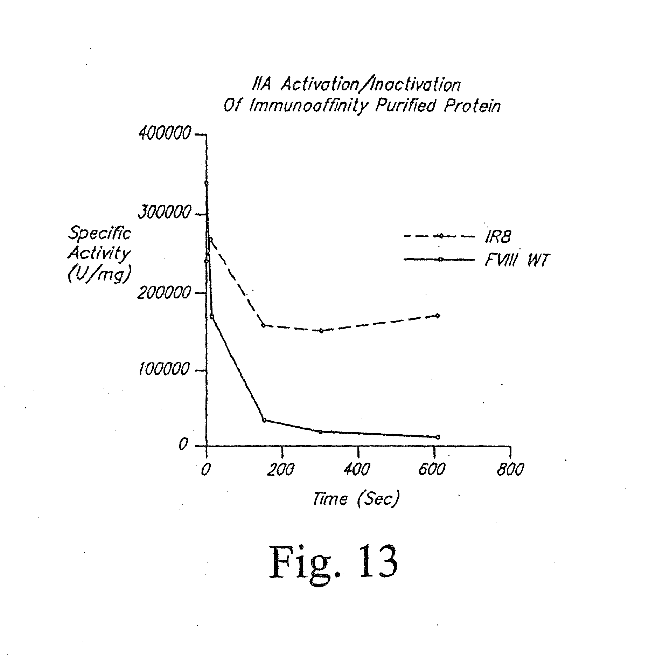

***IR8 demonstrates increased FVIII specific activity in vitro.*** Immunoaffinity purified FVIII WT and IR8 were assayed for FVIII activity utilizing a standard one stage APTT clotting assay, wherein the first time point was 10 seconds. Antigen determinations were made utilizing a FVIII light chain based ELISA. Figure 13 shows the activation and reduced rate of inactivation expressed as specific activity. The specific activity values for IR8 were calculated based on a correction for its molecular weight. IR8 was observed to have a 5-fold increased specific activity compared to FVIII WT (102 ± 43 versus 18.6 ± 7.4 U/mg of protein).

### EXAMPLE 4

### CHARACTERIZATION OF THE INTRAMOLECULAR PROTEIN-PROTEIN INTERACTIONS STABILIZING FVIIIa

### INSTABILITY OF FVIIIA LEADS TO ONE-STAGE/TWO-STAGE ACTIVITY DISCREPANCY

### Experimental Procedures

To demonstrate how instability of FVIIIa leads to one-stage/two-stage (1-st/2-st) activity discrepancy, a modification of the chromogenic two-stage assay was used. In particular, an analysis of the proteins with increasing duration of incubation during the first stage of the assay was performed.

### Results

Wild-type FVIII continued to generate increasing amounts of FXa throughout 16 minutes of the first stage incubation. However, the R531 H, A284E and S289L could generate no more FXa after 8 and 16 minutes than that observed at 4 minutes, consistent with increased rate of inactivation of the mutant FVIIIa molecules early within the first stage of the assay.

### Mutations within A2-A3 Subunit Interface Exhibit One-Stage/Two-Stage Activity Discrepancy

### Experimental Procedures

Mutations within the predicted A2-A3 subunit interface that exhibit similar 1-st/2-st activity discrepancy were also assessed. Missense mutations N694I, R698L and R698W were expressed within a B-domainless FVIII vector by transient expression in COS-1 cells. Each of the mutations resulted in a secreted protein with 1-st/2-st activity discrepancy similar to that reported from patient plasmas.

### Results

Upon thrombin cleavage, purified R698L and R698W proteins exhibited, respectively, twofold and threefold increased rate of A2 subunit dissociation, compared to a B domainless FVIII control, as analyzed in an optical biosensor. Thus, these mutations along the predicted A2-A3 subunit interface exhibit the same molecular mechanism of increased instability of FVIIIa as those mutations described along the A1-A2 interface. This suggests that the entire tightly packed hydrophobic core within the predicted pseudo-threefold axis contributes to stabilization of FVIIIa. Pipe, S.W. et al., Blood 97:685-691 (2001) and Pipe, S.W. et al., Poster presentation at Congress of the International Society on Thrombosis and Haemostasis, Paris, France, July 6-12 (2001), both of which are incorporated herein by reference.

### Stabilization of a Functional Form of FVIIIa by a Strategically Placed Disulfide Bond Experimental Procedures

***Generation* of *COS-1 cell lines for in vitro analysis.*** Cysteine mutations were introduced into each of the following sites: ^{CYS}282, ^{CYS}284 and ^{CYS}531 separately through oligonucleotide-directed mutagenesis and expressed the mutant plasmids in COS-1 cells for *in vitro* analysis. Each of the mutants were expressed successfully and active. Two complementary cysteine mutations were then introduced into both the A1 and A2 subunits. It is believed that the sulfhydryl groups from either ^{CYS}282 or ^{CYS}284 were close enough to potentially form a disulfide bond with the sulfhydryl group of ^{CYS}531. Standard protein analysis techniques were used to demonstrate the presence of a disulfide bond between the resulting A1 and A2 subunits.

It has been shown that an A2-A3 disulfide bond may be obtained based on a molecular model of the A domains of FVa (Pellequer et al., Thrombosis Haemostatis, 84:849-57 (2000)), indicating that the molecular model could not predict which cysteine mutations would work, as only one successful disulfide bond resulted from several strategies attempted.

### Results

Without being bound by theory, it is believed that the introduction of a disulfide bond stabilizes the A2-A3 interaction, and may thereby increase the affinity of A2 with the A1/A3-C1-C2 heterodimer. Cysteine mutations are made at residues predicted to be adjacent in the model and as suggested from studies of the hemophilia point mutations at ^{ASN}694, ^{ARG}698 and ^{MET}1947. Mutation at the A2-A3 interface is thought to have a less disturbing structural effect on the FVIII and enable more efficient expression for detailed analysis.

### EXAMPLE 5

### Increase of the plasma stability of FVIIIa in vivo

### Experimental Procedures

***Methods**.* Three FVIII mutants were prepared in which amino acid(s) were changed to the homologous residues of FV, according to the methods described herein. Mutants were expressed in COS cells and protein purified by immunoaffinity chromatography.

### Results

***Reduction in specific activity.*** Mutants M/F 2199/2200 W/W, L/L 2251/2252 L/S (L2252S), and M/F/L 2199/2200/2252 W/W/S had specific activity in the range of 90-180% of wild type FVIII in both 1-stage and 2-stage commercial aPTT assays that contain a large excess of PL. In a PL-limiting Xase assay (sonicated vesicles of PS:PE:PC 4:20:76, 0.15 µM PL) the mutants had >95%, >95%, and 85% reduction, respectively, in specific activity. Phospholipid titration indicated that maximum activity for the mutants occurred at concentrations of 800, 800, and 200 µM versus 1 µM for wild type FVIII. In a Xase assay with saturating PL, 1000 µM, the apparent affinity of factor IXa for the mutants was decreased approximately 4-fold for the three mutants and the maximum catalytic rate decreased by approximately 50, 80, and 50%, respectively. When the PS content of was increased from 4% to 15% PS, all three mutants supported Xase activity within 60% of wild type FVIII although the apparent affinity for factor IXa was reduced 5-fold.

***Specific interactions with phospholipid and Factor IXa**.* Together these results indicate that the hydrophobic spikes composed of M/F 2199/2200 and L/L 2251/2252 have specific interactions with both phospholipid and factor IXa that are distinct from those of the homologous residues of factor V. Equal or increased activity of M/F/L 2199/2200/2252 W/W/S versus either mutant, in which a single hydrophobic pair was altered, suggests that the two hydrophobic pairs may interact cooperatively in the presence of PL and factor IXa. See Gilbert GE, et al. J. Biol. Chem., in press (2002); Gilbert, GE, et al. Oral presentation at the annual meeting of the American Society of Hematology, Orlando, FL, Dec. 10 (2001); and Saenko E.L. et al., VOX SANG, in press (2002), all of which are incorporated herein by reference.

### EXAMPLE 6

### INDUCIBLE vWF-BINDING OF INACTIVATION RESISTANT FACTOR VIII Experimental Procedures

Immulon 2 microtiter wells (Dynatech Laboratories, Inc., Chantilly, VA) were coated with FVIII antibody at a concentration of 2 ìg/ml overnight at 4°C in a buffer of 0.05 M sodium carbonate/bicarbonate pH 9.6. Wells were washed with TBST (50 mM Tris HCL/pH 7.6, 150 mM NaCl, 0.05% Tween 20) then blocked with 3% bovine serum albumin (BSA) in TBST. Protein samples were diluted in TBST, 3% BSA, 1% factor VIII-deficient human plasma +/-ESH8 (molar ratio of ESH8:FVIII protein = 2:1). Samples were incubated for 2 hours at 37°C in 1.7 ml microfuge tubes. Samples were then incubated for an additional 2 hours in the blocked and washed microtiter wells. Wells were then washed in TBST containing 10 mM CaCl₂. Anti-vWF-HRP antibody was diluted in TBST, 3% BSA, 10 mM CaCl₂ and incubated in the wells for 2 hours at 37°C. Following additional washing with TBST containing 10 mM CaCl₂, OPD substrate was added to the wells and incubated for 3 minutes. The color reaction was stopped with 2 M H₂SO₄ and the optical density (O.D.) read at 490 nm using an EL 340 automated microplate reader (Biotek Instruments Inc., Winooski, VT).

### Results

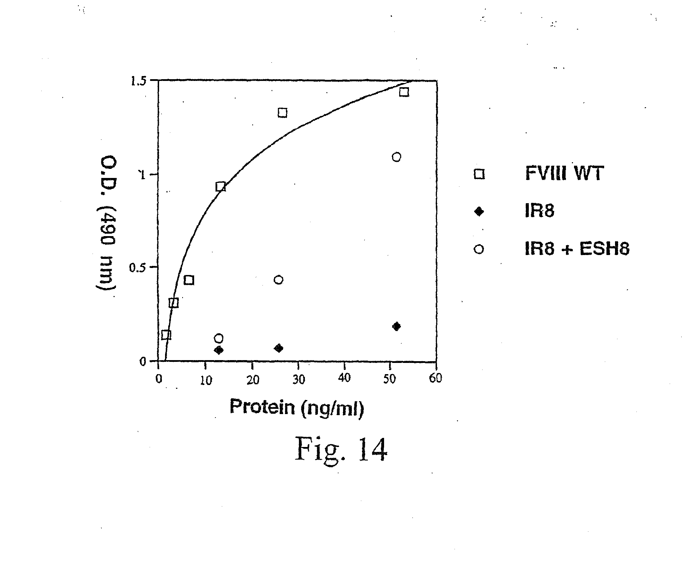

***FVIII-vWF binding.*** Figure 14 shows the results of the FVIII-vWF binding ELISA. An anti-A2 domain trap was used. After a 4 hour incubation with FVIII-deficient plasma (1:100 dilution), binding was detected by perioxidase conjugated anti-vWFab. As shown in Figure 14, a 10-fold lower binding affinity of IR8 to vWF is observed in the absence of ESH8 compared to wild-type FVIII, and a 2-fold lower binding affinity is observed in the presence of ESH8.

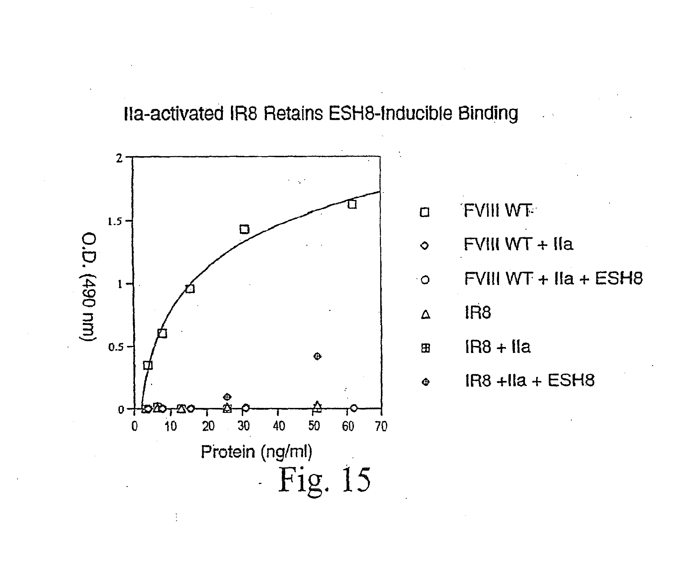

Figure 15 shows the results of the FVIII-vWF binding ELISA with thrombin (IIa) and/or ESH8. The same ELISA method was used however a 2-fold molar excess of ESH8 was employed as well as a 4 hour incubation with IIa (1 U/ml) in the presence of FVIII deficient plasma. As shown in Figure 15, IR8 retains activity for vWF after thrombin activation suggesting that the heterodimer is intact after thrombin cleavage and ESH8 stabilizes the light chain confirmation such that it retains some affinity for vWF.

Since the binding assays described above utilize a "trap" antibody that only recognizes the A2-domain of FVIII, it will only detect FVIII-vWF complexes that recognize the A2-domain in association with the rest of the protein. Therefore, following the 4 hour incubation of the protein in the presence of excess thrombin, FVIII wild-type will not only have been fully activated but it will have also have been completely inactivated through A2 dissociation and/or further proteolytic cleavages, and will no longer associate with vWF in a complex that will be recognized by this assay. The inactivation resistant FVIII of the present invention thus retains inducible binding even following complete activation by thrombin.

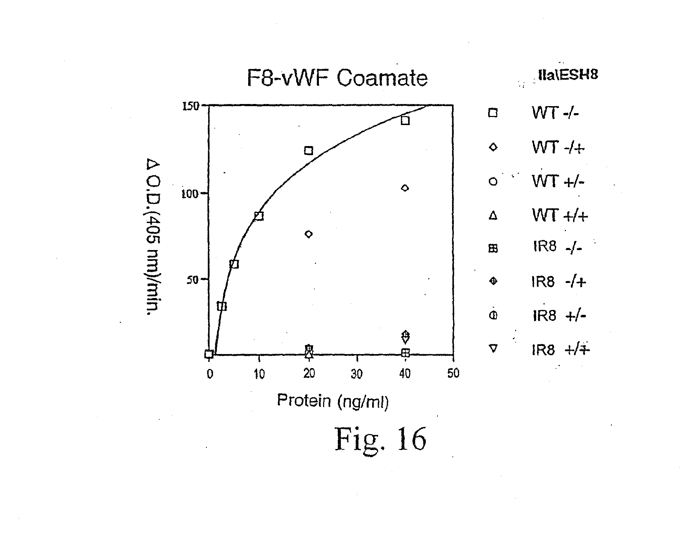

It was also shown that the inducible vWF-binding form of the inactivation resistant FVIII of the present invention retained activity. In this assay, an anti - vWF antibody was used as the "trap" for the ELISA. The same incubation was performed in the presence and absence of thrombin and ESH8. Following immobilization of the FVIII-vWF complex on the plate, FVIII activity was measured using a chromogenic FVIII assay kit (Coamatic, Pharmacia Hepar, Franklin, OH) within the ELISA wells. As shown in Figure 16, following activation by thrombin, no demonstrably active FVIII -vWF complexes were observed for FVIII wild-type. However, the inactivation resistant FVIII still had detectable activity under the same conditions. This suggests that following thrombin activation, the inactivation resistant FVIII is cleaved to a heterodimer of A1 in association with a modified light chain of A2-b-A3-C1-C2 that has ESH8-inducible binding to vWF, and retains FVIII activity.

The functional impact of this ESH8-induced IR8-vWF complex was also evaluated by assaying for FVIII activity via APTT (Table 3). In the absence of ESH8, immunoaffinity purified FVIII WT and IR8 demonstrated minimal loss of activity over a 4 hour incubation at 37°C with FVIII-deficient plasma. In the presence of ESH8, FVIII WT activity was inhibited by approximately 70%, whereas IR8 retained 100% of its initial activity. These results suggest that inactivation of WT FVIII in the presence of ESH8 may be due to A2 subunit dissociation and IR8 is resistant to inactivation by ESH8 because it is not susceptible to A2 subunit dissociation.

**TABLE 3**

| **ESH8 Does Not Inhibit IR8 Activity In Presence Of vWF** | | |
|---|---|---|
| **Protein** | **% Of Initial Activity** | |
| | - ESH8 | + ESH8 |
| FVIII WT | 92 ± 3 | 29 ± 13 |
| IR8 | 101 ±2 | 120 ± 27 |

### EXAMPLE 7

### AFFINITY AND ACTIVITY OF IR8

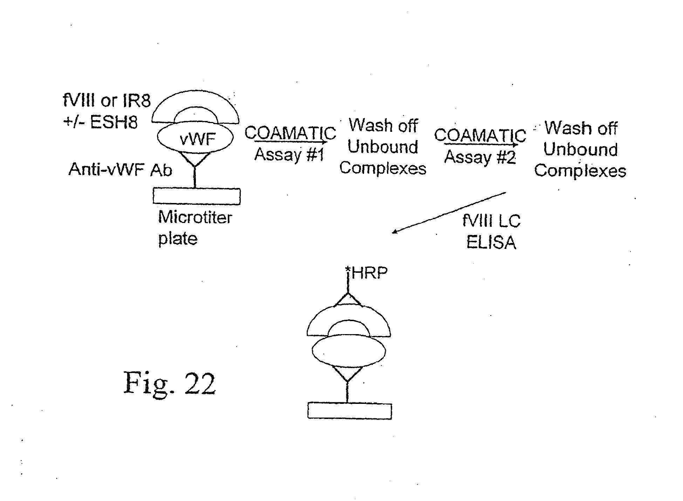

***IR8 Affinity for von Willebrand factor and phospholipid.*** ELISA and affinity biosensor analysis demonstrated IR8 had a 20-fold reduced affinity for von Willebrand factor (vWF), but a 34-fold increased affinity for phospholipid (PL) compared to rFVIII. These changes were attributed to deletion of the AR. In contrast to wild-type FVIII, these affinities were not changed upon thrombin activation of IR8. The monoclonal antibody ESH8 increases the affinity of the thrombin-cleaved FVIII LC to vWF by preventing a LC conformational change that follows proteolytic removal of the AR *in vitro* (Figure 22).

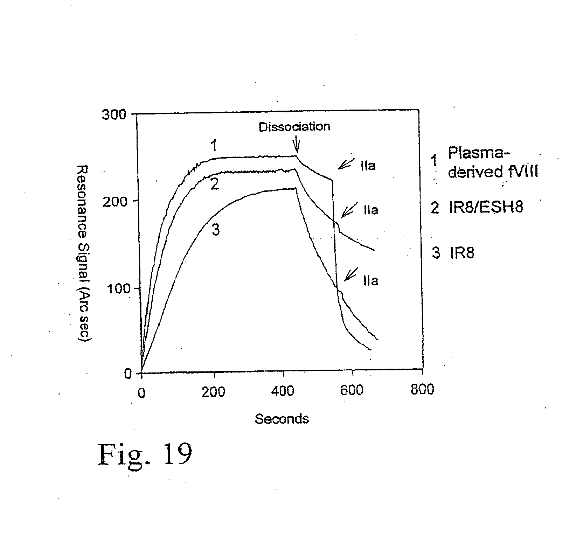

It was proposed that ESH8 inhibits FVIII activity *in vitro* by reducing the rate of vWF dissociation from FVIII upon thrombin activation. However, a complex of IR8/ESH8 demonstrated increased affinity for vWF *in vitro* (IR8 versus rFVIII, K_{d}=1.3 nM versus 0.3 nM), while retaining full activity bound to vWF. Anti-FVIII antibodies specific for the PL binding site were still able to bind, suggesting that the PL binding site and the vWF binding site do not overlap within this LC conformation. Moreover, in contrast to FVIII WT, thrombin activation of IR8/ESH8 does not alter vWF dissociation (Figure 19).

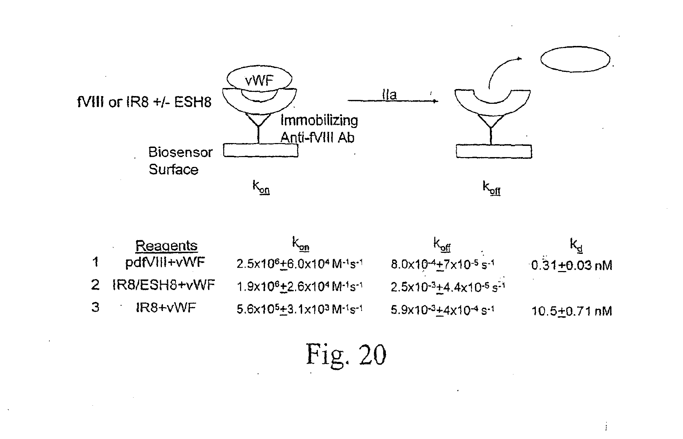

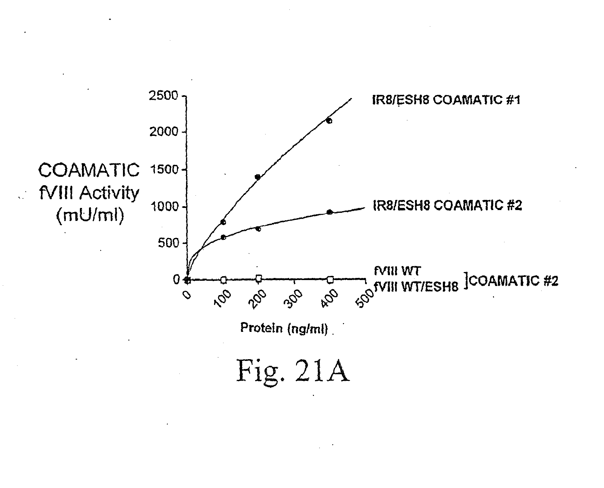

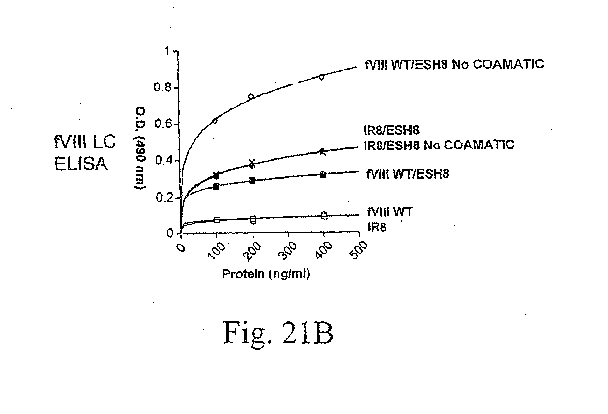

***Kinetics of IR8 and vWF.*** The kinetics of IR8-vWF association and dissociation are set forth in Figure 20. The kinetics of thrombin activation of IR8 shows a loss of activity within the first 30 seconds and then remains stable at approximately 40% of peak activity for several hours (Pipe, S.W. et al., PNAS (USA) 94(22):11851-6 (1997)). The difference in the activity of IR8 between COAMATIC #1 and #2 is consistent with this observation (Figure 21). The post-COAMATIC ELISA confirms that IR8/ESH8 is retained in complex with vWF throughout the assay. Because the ELISA detects LC, FVIII/ESH8 is detected partially complexed with vWF in an inactive form, which may be due to A2 subunit dissociation or the PL binding site is blocked while the FVIII LC is bound to vWF.

Although the affinity of IR8 for vWF is greater than 10-fold lower than FVIII WT, ESH8 induces an IR8-vWF interaction similar to FVIII WT that does not change upon thrombin activation. These results suggest that ESH8 induces a conformation of the LC that retains high affinity for vWF that is independent of the presence of the AR. Without being bound by theory, the AR may be responsible for regulating FVIII cofactor activity as the presence of the AR induces a high affinity vWF binding LC conformation and blocks that PL binding site and the absence of the AR results in a LC conformation that has low affinity for vWF thus the PL binding site is not blocked.

### IR8-vWF Interaction Is Not Blocked By Mab NMC-VIII/5 Experimental Procedures

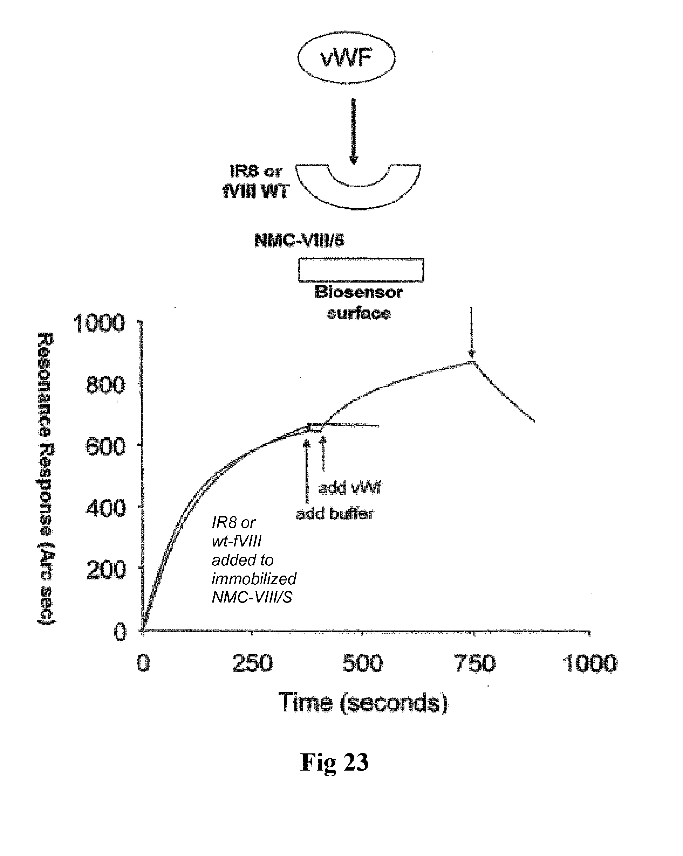

30 nM of IR8 or FVIII WT were added to Mab NMC-VIII/5 immobilized at 10 ng/mm². Following immobilization, the ligand was replaced by buffer at the first arrow and then after 30 sec, vWF was added (first arrow) at 10 nM (Figure 23).

### Results

No signal was observed for vWF binding to immobilized FVIII WT, indicating NMC-VIII/5 completely blocks the vWF binding site. In contrast, vWF binds to IR8 captured on NMC-VIII/5 (kₒₙ=1.4x10⁵ M⁻¹s⁻¹, k_{off}=4.2x10⁻³ s⁻¹, k_{d}=29.6 nM). At the second arrow, dissociation of vWF from IR8 was initiated (Figure 23). The rate of spontaneous dissociation of IR8 or FVIII WT from NMC-VIII/5 is negligible (Figure 23).

### Increased concentrations of vWF Does Not Inhibit Binding of IR8/ESH8 Complexes to Phospholipids

### Experimental Procedures

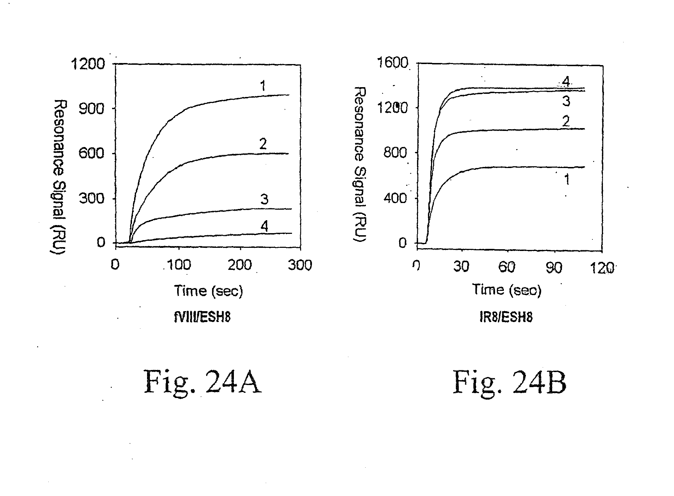

SPIII is a 340 kDa homodimeric disulfide-linked vWF fragment (residues 1-1365 of vWF) and has affinity for FVIII similar to intact vWF. Saenko, E.L. et a/., J. Biol. Chem. 270:13826-13833 (1995). The effect of the increasing concentrations of SPIII (no SPIII in curve 1, 10 nM SPIII in curve 2, 25 nM SPIII in curve 3, 50 nM SPIII in curve 4) on binding of FVIII/ESH8 complex to PSPC monolayer is set forth in Figure 24.

### Results

The increasing concentrations of SPIII progressively inhibited binding of FVIII/ESH8 (10 nM) to PSPC (Figure 24, Panel A). The effect of SPIII was similar to that of vWF added at the same concentration (assuming a molecular weight of 270 kDa per vWF monomer). In contrast, preincubation (30 min, room temperature, HBS, 5 mM CaCl₂) of IR8/ESH8 (3.2 nM) with the increasing concentrations of SPIII fragment lead to increase of the plasmon resonance signal observed when the mixture was added to the PSPC monolayer (Figure 24, Panel B). Upon increase of SPIII concentration (to 50 nM) the signal increased saturation at the level approximately 2-fold greater than that in the absence of SPIII. This is consistent with formation of the IR8/ESH8/SPIII complex (1:1:1) which binds to the PSPC monolayer.

### Determination of the Binding Affinity of IR8/ESH8/SPIII Complex to Phospholipids Experimental Procedures

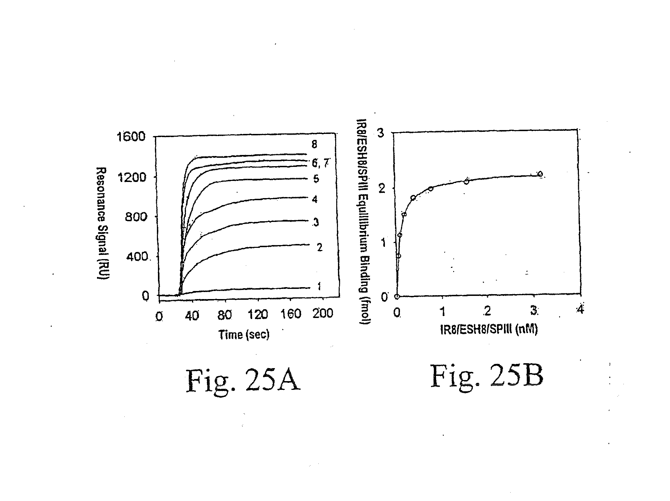

***Preparation of the IR8*/*ESH8*/*SPIII complex.*** The IR8/ESH8/SPIII complex was prepared by incubation (30 min, RT) of 200 nM SPIII, 200 nM ESH8 with varying concentrations of IR8 (0.1 nM-6.4 nM). Association of IR8/ESH8/SPIII with PSPC (25/75) was measured in HBS, 5 mM CaCl₂ until equilibrium was approached. The concentration of the IR8/ESH8/SPIII complex corresponding to curves 1-8 are 0, 0.1, 0.2, 0.4, 0.8, 1.6, 3.2 and 6.4 nM, respectively (Figure 25, Panel A).

***K_{d} value**.* Determination of the K_{d} value for IR8/ESH8/SPIII binding to PSPC monolayer is set forth in Panel B of Figure 25. The open symbols are the values of equilibrium binding (Bₑ) determined from curves 1-7. The solid line shows the best fit of the Be values to the equation: Rₑ=RₘₐₓF/(K_{d}+F), describing equilibrium binding. In the equation, F is the concentration of unbound ligand, Rₘₐₓ - maximal binding capacity of the PSPC monolayer.

### Results

The K_{d} value determined for binding of IR8/ESH8/SPIII complex to PSPC monolayer is 0.286±0.022 nM and similar to that for IR8/ESH8 binding to PSPC (0.242 nM), indicating that SPIII does not significantly affect affinity of IR8/ESH8 for PSPC.

### EXAMPLE 8

### PLASMA PHARMACOKINETICS AND EFFICACY OF IR8 AND IR8/ESH8 COMPLEX IN ANIMALS

### METHODS IN USE

The plasma pharmacokinetics and efficacy of IR8 and IR8/ESH8 complex were evaluated in the Chapel Hill strain of hemophilia A dogs. IR8 protein was produced in Chinese hamster ovary cells and compared to rFVIII (Baxter).

### Experimental Procedures

Hemophilia A dogs were infused with either IR8 or recombinant FVIII (35 units/kg iv) with and without Mab ESH8. Both IR8 and rFVIII corrected the whole blood clotting time (WBCT) to the normal range. A 32% recovery was detected for rFVIII and 11 % for IR8. The plasma half-life was reduced for IR8 compared to rFVIII (2h versus 7h). Both the reduced recovery and short half-life of IR8 relative to rFVIII are consistent with the reduced vWF-binding affinity of IR8.

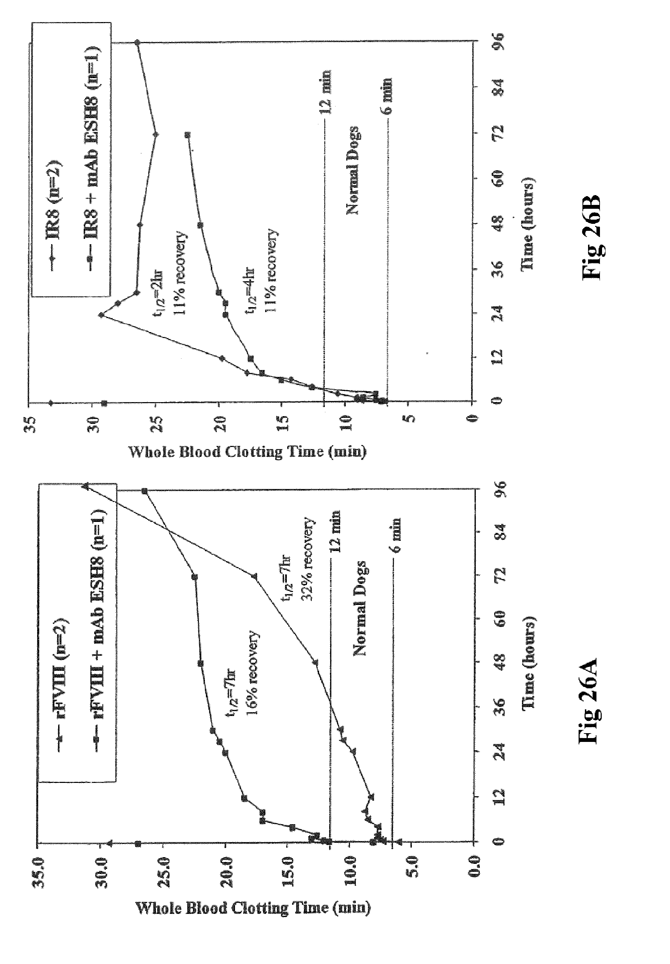

To determine if enhancing the ability of IR8 to complex with vWF would improve its recovery or lengthen the circulating half-life, both IR8 and rFVIII were mixed with the Mab ESH8 at a protein concentration four times greater than the coagulant protein. In the presence of ESH8, the plasma recovery of FVIII WT following infusion was reduced (16%); however, the half-life of clearance was unchanged consistent with inhibition of FVIII WT activity by ESH8 similar to *in vitro* results (Figure 26). In contrast, the half-life of IR8 was doubled to 4 hours in the presence of IR8 with no reduction in the plasma recovery, consistent with stabilization in the plasma through increased binding to vWF, but no inhibition of cofactor activity (Figure 26).

Significantly, IR8 corrected the secondary cuticle bleeding time in the hemophilia A dogs to the normal range in both the presence and absence of ESH8 showing no inhibition of cofactor activity *in vivo.*

### Results

Plasma recovery and clearance were monitored by COAMATIC assay, whole blood clotting times, and ELISA. Plasma recovery of IR8 was reduced (11% versus 32%) and the plasma half-life (t_{1/2}) was significantly shorter (2 h versus 7 h) than rFVIII. These results are consistent with a lack of vWF binding to IR8 *in vivo* and are comparable to the t_{1/2} of FVIII infused into patients with vWF deficiency. Despite this, IR8 was still able to correct the cuticle bleeding time (CBT), similar to rFVIII. IR8/ESH8 complex was prepared by incubating purified IR8 with a 4-fold excess of ESH8. The recovery of IR8 in this complex measured by activity and ELISA assay was still reduced at 11 % but the plasma t_{1/2} was doubled to 4 hours consistent with increased stabilization through binding to vWF. The IR8/ESH8 complex also corrected the CBT, indicating that IR8/ESH/vWF complex may be active *in vivo.*

These results are consistent with ESH8 inducing a LC vWF-binding conformation within IR8 that is similar to intact FVIII LC. However, in sharp contrast to rFVIII, this IR8 LC conformation allows simultaneous high affinity PL binding and does not interfere with cofactor activity (Figure 26).

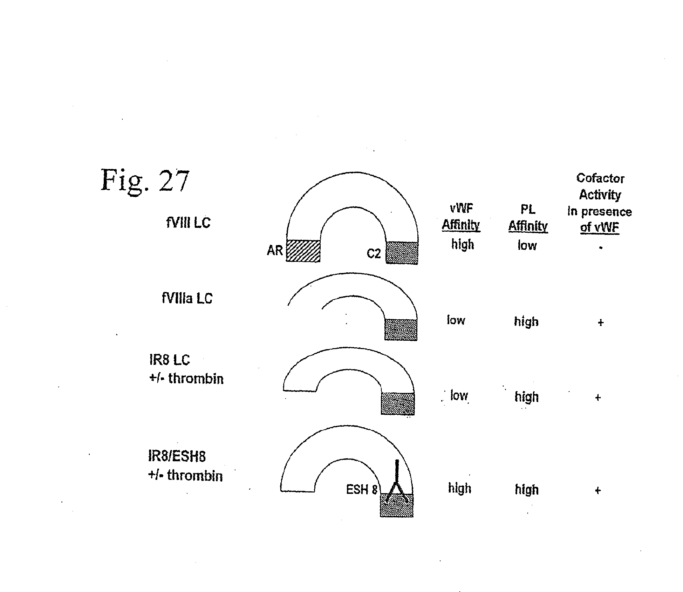

In summary, upon removal of the AR, there is a FVIII LC conformation that retains high vWF and PL binding affinity (Figure 27). The results also demonstrate that the vWF and PL binding sites are not overlapping and competitive in all FVIII LC conformations. The IR8/ESH8 complex has a unique LC confirmation that retains both high affinity vWF and PL affinity. Moreover, the IR8/ESH8/vWF complex is stable and active both *in vitro* and *in vivo.*

### EXAMPLE 9

### FVIII B DOMAIN MUTANTS SHOW INCREASED SECRETION PROPORTIONATE TO THEIR N-LINKED OLIGOSACCHARIDE CONTENT

### Experimental Procedures

**Preparation of FVIII mutants**. FVIII wild-type (intact B domain) and a full B domain-deletion molecule were used as controls. Since FVIII is stabilized in conditioned medium through binding to vWF, all of the FVIII mutants were initially prepared within a BDD-FVIII vector that has no light chain acidic region (90/73) and therefore markedly reduced affinity for vWF. Thus, any improvement in FVIII recovered from the conditioned medium could be more easily attributed to increased rate of secretion. Increasing lengths of B domain sequences were introduced into 90/73 that all started with amino acid (aa) residue 741 of FVIII. Each incremental increase in the size of the B domain included one additional N-linked glycosylation site. The resultant proteins were expressed by transient transfection in COS-1 cells. The relative rates of secretion were determined by FVIII ELISA of the conditioned medium collected from 36 to 60 hours post-transfection.

The N-linked glycosylation sites were then mutated (to glutamine) within the 117 amino acid B domain containing construct (which has 3 putative N-linked oligosaccharides), and the relative rates of secretion were determined as before. This experiment was also repeated with constructs that contained the LC acidic region. Because vWF is limited in serum-containing medium, the same experiment was performed by co-transfection of a vWF expression vector along with the FVIII mutants.

### Results

***Increased secretion.*** All expressed proteins were synthesized efficiently and retained high specific activity that was comparable to their relative secretion. Average secretion of 90/73 was 7.9 ng/ml and that of FVIII wild-type was 62 ng/ml. Increasing segments from the amino-terminal end of the B domain improved FVIII secretion as follows: 29 amino acids, 1.7-fold; 54 amino acids, 3.4-fold; 117 amino acids, 5.3-fold; 163 amino acids, 8.5-fold; and 226 amino acids, 10.8-fold (see Figure 28). Thus, with increasing size of the B domain, and therefore, the number of glycosylation sites, there was an ~10-fold increased secretion.

Compared to the native 117 amino acid B domain construct (5.3-fold increased secretion compared to 90/73), mutation of one N-linked site reduced secretion to 4.5-fold and mutation of 2 N-linked sites reduced secretion to 2.4-fold. Therefore, despite no change in the size of the B domain spacer, decreased oligosaccharide content reduced secretion. When this experiment was repeated with constructs that contained the LC acidic region, a blunted response was observed with only a 2-3 fold increase in secretion. Therefore, the same experiment was performed by co-transfection of a vWF expression vector along with the FVIII mutants. The results demonstrated a similar pattern of increasing FVIII activity up to 10-fold recovered from the medium as the number of N-linked glycosylation sites increased.

Maximal secretion was observed with a 226 amino acid B domain and 6 N-linked oligosaccharides (Figure 29). A non-native B domain did not facilitate increased secretion despite dense N-linked glycosylations (LAMP) (Figure 29).

***B domain mediated interaction.*** Without being limited by theory, it is believed that the B domain, by virtue of its rich oligosaccharide content, mediates interaction with ERGIC-53 to facilitate its ER to Golgi transport. BDD-FVIII has been used in most hemophilia A gene therapy strategies as the full-length cDNA is too large for most viral vectors. These results suggest that addition of N-linked glycosylation sites can improve the secretion of BDD-FVIII up to 10-fold and may increase FVIII expression *in vivo.*

***Structure and function of B Domain**.* Further experimentation was performed to evaluate the impact of density and/or orientation of the oligosaccharides. Two densely glycosylated protein segments derived from the unrelated glycoprotein LAMP-1 (containing either 5 or 9 N-linked oligosaccharides) were substituted for B domain sequence, but did not improve secretion compared to BDD-FVIII. This suggests that the density and/or orientation of the oligosaccharides may be important.

### EXAMPLE 10

### CHARACTERIZATION AND ANALYSIS OF FVIII B-DOMAIN MUTANTS Experimental Procedures

A FVIII B-domain mutant (also referred to herein as the "90/80/b226N6 variant" or "b226N6 variant") includes the Phe309Ser mutant and the b226N6 B-domain variant. In particular, in one embodiment, a FVIII B-domain mutant comprises 226 amino acid B-domain with 6 consensus sites for N-linked glycosylation.

### Results

The FVIII B-domain mutant achieves maximal expression in COS cell and CHO cell transient expression. The secreted protein yields FVIII with high specific activity and is secreted as a single chain without intracellular processing.

The results demonstrated that the hybrid FVIII molecule yields a 15-fold greater expression as compared to BDD-FVIII, while the Phe309Ser mutation alone shows a 6-fold increase compared to BDD-FVIII and the b226N6 mutation alone shows an 8-fold increase in expression compared to BDD-FVIII.

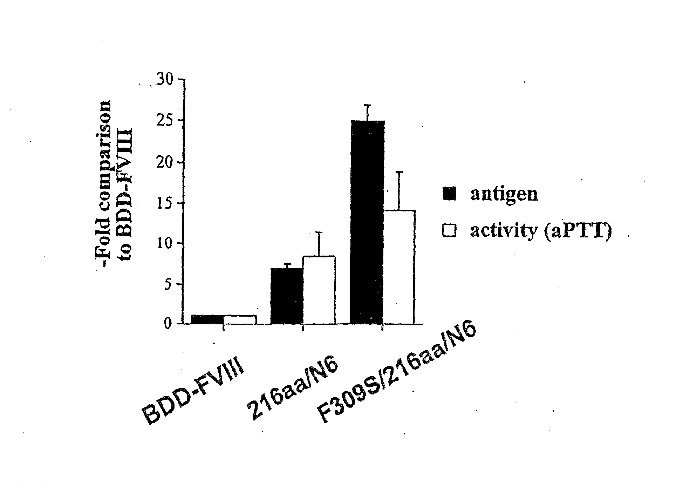

The results further demonstrated that the secretion efficiency of a FVIII construct containing a mutant B domain 226aa/N6 is further enhanced with the point mutation F309S (Figure 30).

### EXAMPLE 11

### EXPRESSION OF BIOENGINEERED FVIII IN VIVO

### Experimental Procedures

A FVIII knockout mouse model of hemophilia A was utilized to analyze the *in vivo* expression of the FVIII molecules of the present invention.

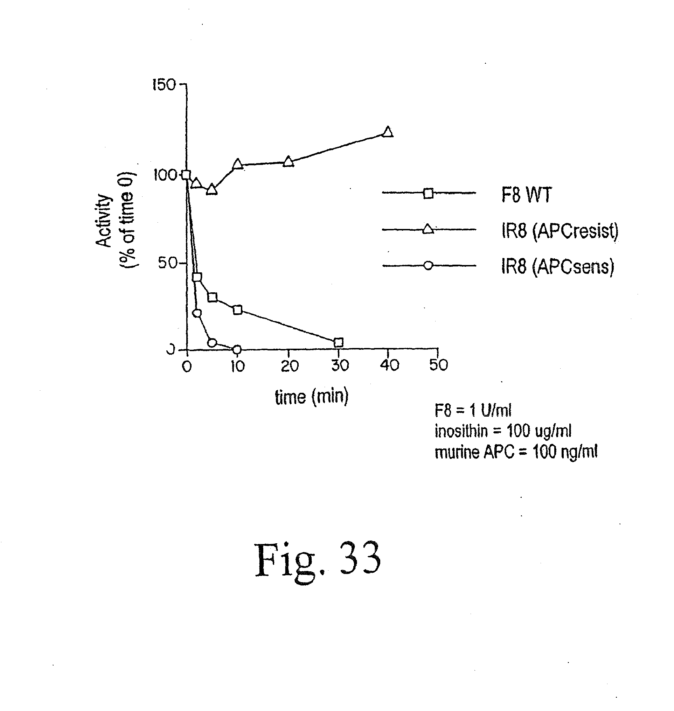

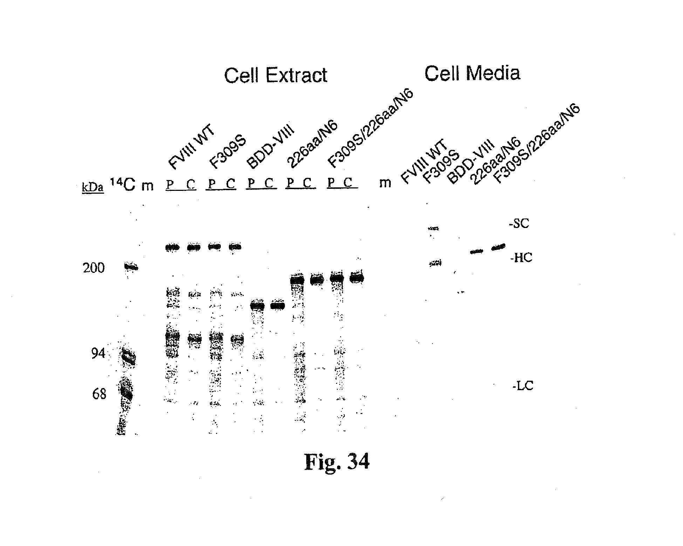

***Methods**.* Plasmid DNA (100 µg) was diluted in 2.5 ml of lactated Ringer's and infused over 10 seconds into the tail vein. Orbital blood collection was performed at 24 and 48 hours and FVIII secretion analyzed by a human FVIII-specific ELISA. The FVIII anitigen and activity were measured in blood (Figures 31 and 33). Figure 34 confirms the presence of 226aa/N6 and F309S/226aa/N6 in the cell media.

### Results

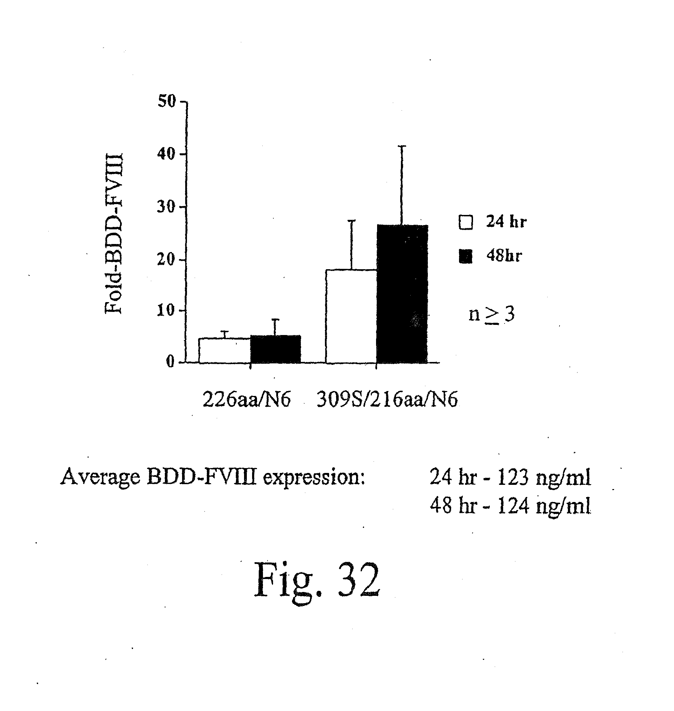

Figures 31 and 32 indicate increased expression of FVIII B domain variants in hemophilia A mice following hydrodynamic tail vein injection of the F309S/226aa/N6 construct. In particular, the 309S/226aa/N6 variant showed increased expression at 48 hours as compared to the 226aa/N6 variant (Figure 32). The data derived indicated that the average BDD-FVIII expression was 123 ng/ml after 24 hours and 124 ng/ml after 48 **hours (see** **Figure 32****).**

### EXAMPLE 12

### PHARMACEUTICAL COMPOSITIONS AND USE

### Pharmaceutical Composition

The FVIII proteins of the present invention can be formulated into pharmaceutically acceptable compositions with parenterally acceptable vehicles and excipients in accordance with procedures known in the art. The pharmaceutical compositions of this invention, suitable for parenteral administration, may conveniently comprise a sterile lyophilized preparation of the protein which may be reconstituted by addition of sterile solution to produce solutions such as isotonic with the blood of the recipient. The preparation may be presented in unit or multi-dose containers, *e.g*. in sealed ampoules or vials.

Such pharmaceutical compositions may also contain pharmaceutically acceptable carriers, diluents, fillers, salts, buffers, stabilizers, and/or other materials well known in the art. The term "pharmaceutically acceptable" means a non-toxic material that does not interfere with the effectiveness of the biological activity of the active ingredient(s). The characteristics of the carrier or other material will depend on the route of administration.

The amount of FVIII protein in the pharmaceutical composition of the present invention will depend upon the nature and severity of the condition being treated, and on the nature of the prior treatments which the patient has undergone. Ultimately, the attending physician will decide the amount of protein with which to treat each individual patient. The duration of intravenous therapy similarly will vary, depending on the severity of the disease being treated and the condition and potential idiosyncratic response of each individual patient.

In addition, the nucleotide sequences encoding the FVIII proteins of the present invention may be associated with a gene therapy delivery system in accordance with procedures known in the art. Such delivery systems include, without limitation, adenoviral, retroviral and adeno-associated viral vectors, as well as liposomes and DNA-protein complexes. The sequences of the present invention are contained in or operatively-linked to such delivery systems in a manner which allows for transcription, e.g., through the use of sufficient regulatory elements. It will be appreciated that a variety of strategies and methodology for creating such gene therapy delivery systems are well known to those skilled in the art.

### Methods of Use

Pharmaceutical compositions containing the proteins of the present invention may be used to treat patients suffering from hemophilia caused by deficiency of FVIII.

In practicing the method of treatment of this invention, a therapeutically effective amount of FVIII protein is administered to a mammal having a hemophiliac condition caused by FVIII deficiency. The term "therapeutically effective amount" means the total amount of each active component of the method or composition that is sufficient to show a meaningful patient benefit, *i.e*. cessation of bleeding.

Administration of the proteins of the present invention can be carried out in a variety of conventional ways. Intravenous administration to the patient may be performed. When administered by intravenous injection, the proteins of the invention will be in the form of pyrogen-free, parenterally acceptable aqueous solutions. A pharmaceutical composition for intravenous injection may contain, in addition to the proteins, an isotonic vehicle such as sodium chloride injection, Ringer's injection, dextrose injection, dextrose and sodium chloride injection, lactated Ringer's injection, or other vehicles as known in the art. The pharmaceutical composition according to the present invention may also contain stabilizers, preservatives, buffers, anti-oxidants, or other additives known to those of skill in the art.

For cutaneous or subcutaneous injection, the proteins of the present invention will be in the form of pyrogen-free, parenterally acceptable aqueous solutions. The preparation of such parenterally acceptable protein solutions, having due regard to pH, isotonicity, stability, and the like, is within the skill in the art.

As with the pharmaceutical compositions containing the proteins of the present invention, gene therapy delivery systems or vehicles containing nucleotide sequences of the present invention may also be used to treat patients suffering form hemophilia caused by deficiency of FvIII. A therapeutically effective amount of such gene therapy delivery vehicles is administered to a mammal having a hemophiliac condition caused by FVIII deficiency. It will be appreciated that administration of the vehicles of the present invention will be by procedures well established in the pharmaceutical arts, e.g. by direct delivery to the target tissue or site, intranasally, intravenously, intramuscularly, subcutaneously, intradermally and through oral administration, either alone or in combination. It will also be appreciated that formulations suitable for administration of the gene therapy delivery vehicles are known in the art and include aqueous and non-aqueous isotonic sterile injection solutions and aqueous and non-aqueous sterile suspensions.

The foregoing discussion discloses and describes merely exemplary embodiments of the present invention. One skilled in the art will readily recognize from such discussion, and from the accompanying drawings and claims, that various changes, modifications and variations can be made therein without departing from the spirit and scope of the invention as defined in the following claims.

All patents and other publications cited herein are expressly incorporated by reference.

Furthermore, the present invention relates to the following items:
1. A method for producing a recombinant Factor VIII protein, comprising the steps of:
   introducing into a cell a nucleic acid molecule encoding a Factor VIII protein operably linked to a promoter, wherein the promoter is characterized by the ability to produce commercially viable Factor VIII protein; and
   incubating the cell under conditions for producing Factor VIII protein.
2. The method of item 1, wherein the cell is a mammalian cell.
3. The method of item 2, wherein wherein the mammalian cell is selected from the group consisting of a COS-1, CHO, and HEK 293 cell.
4. The method of item 1, wherein the nucleic acid molecule comprises a cDNA which encodes the Factor VIII protein.
5. The method of item 1, wherein the nucleic acid molecule is operably linked to a Chinese hamster elongation factor 1-α (CHEF1) promoter.
6. The method of item 1, wherein the Factor VIII protein comprises a deletion of the B-domain starting at Arg 740 when the protein is aligned with the wild-type Factor VIII, followed by an addition of an amino acid spacer containing at least one N-linked glycosylation site, wherein the amino acid spacer containing the at least one N-iinked glycosylation site facilitates the secretion or expression of the B-domain-deletion Factor VIII protein.
7. The method of item 6, wherein the Factor VIII protein comprises an amino acid sequence inserted at position 750 when the protein is aligned with the wild-type Factor VIII, the inserted amino acid sequence consisting of a 226 amino acid spacer containing 6 N-linked glycosylation sites, thereby partially replacing the B domain of the modified Factor VIII protein.
8. The method of item 6, wherein the Factor VIII protein comprises an amino acid sequence inserted at position 769 when the protein is aligned with the wild-type Factor VIII, the inserted amino acid sequence consisting of a 29 amino acid spacer containing one N-linked glycosylation site, thereby partially replacing the B domain of the modified Factor VIII protein.
9. The method of item 6, wherein the Factor VIII protein comprises an amino acid sequence inserted at position 794 when the protein is aligned with the wild-type Factor VIII, the inserted amino acid sequence consisting of a 55 amino acid spacer containing 2 N-linked glycosylation sites, thereby partially replacing the B domain of the modified Factor VIII protein.
10. The method of item 6, wherein the Factor VIII protein comprises an amino acid sequence inserted at position 857 when the protein is aligned with the wild-type Factor VIII, the inserted amino acid sequence consisting of a 117 amino acid spacer containing 3 N-linked glycosylation sites, thereby partially replacing the B domain of the modified Factor VIII protein.
11. The method of item 6, wherein the the Factor VIII protein comprises an amino acid sequence inserted at position 903 when the protein is aligned with the wild-type Factor VIII, the inserted amino acid sequence consisting of a 163 amino acid spacer containing 4 N-linked glycosylation sites, thereby partially replacing the B domain of the modified Factor VIII protein.
12. The method of item 6, wherein the Factor VIII protein comprises an amino acid sequence inserted at position 946 when the protein is aligned with the wild-type Factor VIII, the inserted amino acid sequence consisting of a 206 amino acid spacer containing 5 N-linked glycosylation sites, thereby partially replacing the B domain of the modified Factor VIII protein.
13. The method of item 6, wherein the Factor VIII protein comprises an amino acid sequence inserted at position 1009 when the protein is aligned with the wild-type Factor VIII, the inserted amino acid sequence consisting of a 269 amino acid spacer containing 8 N-linked glycosylation sites, thereby partially replacing the B domain of the modified Factor VIII protein.
14. The method of item 1 wherein the Factor VIII protein comprises a heterologous sequence.
15. A method for identifying a cell expressing commercially viable Factor VIII protein, comprising:
   a) introducing into cells a nucleic acid molecule encoding a Factor VIII protein operably linked to a promoter, wherein the promoter is characterized by the ability to produce commercially viable Factor VIII protein;
   b) incubating the cells under conditions for producing Factor VIII protein;
   c) selecting clones expressing high levels of FVIII relative to the other clones;
   d) recloning the cells selected in step c); and
   e) identifying at least one subclone expressing a higher level of FVIII relative to those selected in step c).
16. A nucleic acid molecule encoding a Factor VIII protein operably linked to a Chinese hamster elongation factor 1-α (CHEF1) promoter.
17. The nucleic acid molecule of item 16, wherein the nucleic acid molelcuule comprises a cDNA which encodes the Factor VIII protein.
18. The nucleic acid molecule of item 16, wherein the Factor VIII protein comprises a deletion of the B-domain starting at Arg 740 when the protein is aligned with the wild-type Factor VIII, followed by an addition of an amino acid spacer containing at least one N-linked glycosylation site, wherein the amino acid spacer containing the at least one N-linked glycosylation site facilitates the secretion or expression of the B-domain-deletion Factor VIII protein.
19. The nucleic acid molecule of item 16, wherein the Factor VIII protein comprises an amino acid sequence inserted at position 750 when the protein is aligned with the wild-type Factor VIII, the inserted amino acid sequence consisting of a 226 amino acid spacer containing 6 N-linked glycosylation sites, thereby partially replacing the B domain of the modified Factor VIII protein.
20. The nucleic acid molecule of item 16, wherein the Factor VIII protein comprises an amino acid sequence inserted at position 769 when the protein is aligned with the wild-type Factor VIII, the inserted amino acid sequence consisting of a 29 amino acid spacer containing one N-linked glycosylation site, thereby partially replacing the B domain of the modified Factor VIII protein.
21. The nucleic acid molecule of item 16, wherein the Factor VIII protein comprises an amino acid sequence inserted at position 794 when the protein is aligned with the wild-type Factor VIII, the inserted amino acid sequence consisting of a 55 amino acid spacer containing 2 N-linked glycosylation sites, thereby partially replacing the B domain of the modified Factor VIII protein.
22. The nucleic acid molecule of item 16, wherein the Factor VIII protein comprises an amino acid sequence inserted at position 857 when the protein is aligned with the wild-type Factor VIII, the inserted amino acid sequence consisting of a 117 amino acid spacer containing 3 N-linked glycosylation sites, thereby partially replacing the B domain of the modified Factor VIII protein.
23. The nucleic acid molecule of item 16, wherein the the Factor VIII protein comprises an amino acid sequence inserted at position 903 when the protein is aligned with the wild-type Factor VIII, the inserted amino acid sequence consisting of a 163 amino acid spacer containing 4 N-linked glycosylation sites, thereby partially replacing the B domain of the modified Factor VIII protein.
24. The nucleic acid molecule of item 16, wherein the Factor VIII protein comprises an amino acid sequence inserted at position 946 when the protein is aligned with the wild-type Factor VIII, the inserted amino acid sequence consisting of a 206 amino acid spacer containing 5 N-linked glycosylation sites, thereby partially replacing the B domain of the modified Factor VIII protein.
25. The nucleic acid molecule of item 16, wherein the Factor VIII protein comprises an amino acid sequence inserted at position 1009 when the protein is aligned with the wild-type Factor VIII, the inserted amino acid sequence consisting of a 269 amino acid spacer containing 8 N-linked glycosylation sites, thereby partially replacing the B domain of the modified Factor VIII protein.
26. The nucleic acid molecule of item 16, which encodes a heterologous protein.
27. The method of item 15 further comprising:
   f) recloning the at least one subclone identified in step e); and
   g) identifying at least one subclone expressing a higher level of FVIII relative to the at least one subclone selected in step e).
28. The method of item 1, wherein the Factor VIII protein comprises a deletion of the B-domain starting at Arg 740 when the protein is aligned with the wild-type Factor VIII.
29. The method of item 1, wherein the Factor VIII protein comprises a substitution of phenylalanine at position 309 to serine when the protein is aligned with the wild-type Factor VIII.
30. The method of item 28, wherein the Factor VIII protein comprises a substitution of phenylalanine at position 309 to serine when the protein is aligned with the wild-type Factor VIII.
31. The nucleic acid molecule of item 16, wherein the Factor VIII protein comprises a deletion of the B-domain starting at Arg 740 when the protein is aligned with the wild-type Factor VIII.
32. The nucleic acid molecule of item 16 wherein the Factor VIII protein comprises a substitution of phenylalanine at position 309 to serine when the protein is aligned with the wild-type Factor VIII.
33. The nucleic acid molecule of item 31 wherein the Factor VIII protein comprises a substitution of phenylalanine at position 309 to serine when the protein is aligned with the wild-type Factor VIII.
34. The method of item 1 wherein the Factor VIII protein is produced at a level seleted from the group consisting of at least about 20 IU/mL, at least about 30 IU/mL, at least about 40 IU/mL, at least about 50 IU/mL, at least about 60 IU/mL, at least about 70 IU/mL, at least about 80 IU/mL, at least about 90 IU/mL, at least about 100 IU/mL, at least about 110 IU/mL, at least about 120 IU/mL, at least about 130 IU/mL, at least about 140 IU/mL, at least about 150 IU/mL, at least about 160 IU/mL, at least about 170 IU/mL, at least about 180 IU/mL, at least about 190 IU/mL, at least about 200 IU/mL, and at least about 210 IU/mL.

## Claims

1. A method for producing a recombinant Factor VIII protein, comprising the steps of:
introducing into a cell a nucleic acid molecule encoding a Factor VIII protein operably linked to a Chinese hamster elongation factor 1-α (CHEF1) promoter,
wherein the promoter is **characterized by** the ability to produce commercially viable Factor VIII protein; and
incubating the cell under conditions for producing Factor VIII protein.

2. The method of claim 1, wherein the cell is a mammalian cell.

3. The method of claim 2, wherein the mammalian cell is selected from the group consisting of a COS-1, CHO, and HEK 293 cell.

4. The method of any one of claims 1 to 3, wherein the Factor VIII protein comprises a heterologous sequence.

5. A method for identifying a cell expressing commercially viable Factor VIII protein, comprising:
a) introducing into cells a nucleic acid molecule encoding a Factor VIII protein operably linked to a Chinese hamster elongation factor 1-α (CHEF1) promoter, wherein the promoter is **characterized by** the ability to produce commercially viable Factor VIII protein;
b) incubating the cells under conditions for producing Factor VIII protein;
c) selecting clones expressing high levels of FVIII relative to the other clones;
d) recloning the cells selected in step c); and
e) identifying at least one subclone expressing a higher level of FVIII relative to those selected in step c).

6. A nucleic acid molecule encoding a Factor VIII protein operably linked to a Chinese hamster elongation factor 1-α (CHEF1) promoter.

7. The nucleic acid molecule of claim 6 or the method of any one of claims 1 to 5, wherein the nucleic acid molecule comprises a cDNA which encodes the Factor VIII protein.

8. The nucleic acid molecule of claim 6 or 7 or the method of any one of claims 1 to 4, wherein the Factor VIII protein comprises
(a) a deletion of the B-domain starting at Arg740 when the protein is aligned with the wild-type Factor VIII, followed by an addition of an amino acid spacer containing at least one N-linked glycosylation site, wherein the amino acid spacer containing the at least one N-linked glycosylation site facilitates the secretion or expression of the B-domain-deletion Factor VIII protein; or
(b) an amino acid sequence inserted at position 750 when the protein is aligned with the wild-type Factor VIII, the inserted amino acid sequence consisting of a 226 amino acid spacer containing 6 N-linked glycosylation sites, thereby partially replacing the B domain of the modified Factor VIII protein; or
(c) an amino acid sequence inserted at position 769 when the protein is aligned with the wild-type Factor VIII, the inserted amino acid sequence consisting of a 29 amino acid spacer containing one N-linked glycosylation site, thereby partially replacing the B domain of the modified Factor VIII protein; or
(d) an amino acid sequence inserted at position 794 when the protein is aligned with the wild-type Factor VIII, the inserted amino acid sequence consisting of a 55 amino acid spacer containing 2 N-linked glycosylation sites, thereby partially replacing the B domain of the modified Factor VIII protein; or
(e) an amino acid sequence inserted at position 857 when the protein is aligned with the wild-type Factor VIII, the inserted amino acid sequence consisting of a 117 amino acid spacer containing 3 N-linked glycosylation sites, thereby partially replacing the B domain of the modified Factor VIII protein; or
(f) an amino acid sequence inserted at position 903 when the protein is aligned with the wild-type Factor VIII, the inserted amino acid sequence consisting of a 163 amino acid spacer containing 4 N-linked glycosylation sites, thereby partially replacing the B domain of the modified Factor VIII protein; or
(g) an amino acid sequence inserted at position 946 when the protein is aligned with the wild-type Factor VIII, the inserted amino acid sequence consisting of a 206 amino acid spacer containing 5 N-linked glycosylation sites, thereby partially replacing the B domain of the modified Factor VIII protein; or
(h) an amino acid sequence inserted at position 1009 when the protein is aligned with the wild-type Factor VIII, the inserted amino acid sequence consisting of a 269 amino acid spacer containing 8 N-linked glycosylation sites, thereby partially replacing the B domain of the modified Factor VIII protein.

9. The nucleic acid molecule of any one of claims 6 to 8, which encodes a heterologous protein.

10. The method of claim 5 further comprising:
f) recloning the at least one subclone identified in step e); and
g) identifying at least one subclone expressing a higher level of FVIII relative to the at least one subclone selected in step e).

11. The method of any one of claims 1 to 4, wherein the Factor VIII protein comprises
(a) a deletion of the B-domain starting at Arg740 when the protein is aligned with the wild-type Factor VIII; and/or
(b) a substitution of phenylalanine at position 309 to serine when the protein is aligned with the wild-type Factor VIII.

12. The nucleic acid molecule of claim 6, wherein the Factor VIII protein comprises
(a) a deletion of the B-domain starting at Arg740 when the protein is aligned with the wild-type Factor VIII; or
(b) a substitution of phenylalanine at position 309 to serine when the protein is aligned with the wild-type Factor VIII.

13. The nucleic acid molecule of claim 12(a) wherein the Factor VIII protein comprises a substitution of phenylalanine at position 309 to serine when the protein is aligned with the wild-type Factor VIII.

14. The method of any one of claims 1 to 4, 7 or 11, wherein the Factor VIII protein is produced at a level selected from the group consisting of at least about 20 IU/mL, at least about 30 IU/mL, at least about 40 IU/mL, at least about 50 IU/mL, at least about 60 IU/mL, at least about 70 IU/mL, at least about 80 IU/mL, at least about 90 IU/mL, at least about 100 IU/mL, at least about 110 IU/mL, at least about 120 IU/mL, at least about 130 IU/mL, at least about 140 IU/mL, at least about 150 IU/mL, at least about 160 IU/mL, at least about 170 IU/mL, at least about 180 IU/mL, at least about 190 IU/mL, at least about 200 IU/mL, and at least about 210 IU/mL.
